# EUROPEAN PATENT APPLICATION

(11) **EP 1 090 920 A1**
(43) Date of publication of application: **11.04.2001**
(21) Application number: 99926786.7
(22) Date of filing: 24.06.1999
(51) Int. Cl.: C07D 501/59, C07D 519/00, A61K 31/545

(54) **CEPHEM COMPOUNDS**

(30) Priority: 24.06.1998 JP 17788098
(71) Applicant: ZENYAKU KOGYO KABUSHIKI KAISHA, Tokyo 103-0022 (JP)
(72) Inventor: HANAKI, Hideaki, Hiratsuka-shi, Kanagawa 259-1217 (JP); YAMAZAKI, Hiroaki, Kenkyusho Zenyaku Kogyo K. K., Nerima-ku, Tokyo 178-0062 (JP); TSUCHIDA, Yoshio, Kenkyusho Zenyaku Kogyo K. K., Nerima-ku, Tokyo 178-0062 (JP); SATO, Hideki, Kenkyusho Zenyaku Kogyo K. K., Nerima-ku, Tokyo 178-0062 (JP); HIRAMATSU, Keiichi, Minato-ku, Tokyo 108-0074 (JP); KAWASHIMA, Seiichiro, Kenkyusho Zenyaku Kog. K.K., Nerima-ku, Tokyo 178-0062 (JP)
(74) Representative: LOUIS, PÖHLAU, LOHRENTZ & SEGETH
(86) International application number: JP9903367
(87) International publication number: WO9967256

(57) **Abstract**

Cephem derivatives or pharmaceutically acceptable salts thereof represented by the formula I: 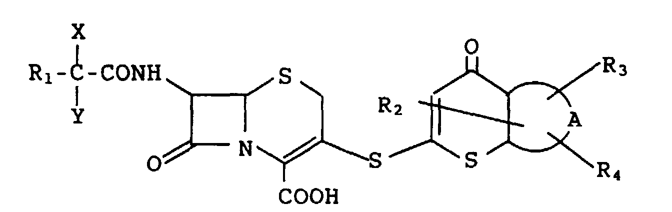 wherein 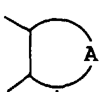 represents benzene ring, pyridine ring, pyrazine ring or 5-membered aromatic heterocycle (having one oxygen or sulfur atom as ring-constituting atom);
X and Y respectively represent hydrogen atoms or CXY represents C=N-OH;
R₁ represents phenyl, thienyl or thiazolyl (which may be substituted with amino group or halogen atom); and
R₂, R₃ and R₄ respectively represent hydrogen atoms, halogen, hydroxy C₁-C₆ alkyl, isothiuronium C₁-C₆ alkyl, amino C₁-C₆ alkyl or amino C₁-C₆ alkylthiomethyl, there being no R₄ where 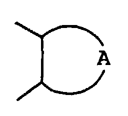 represents 5-membered aromatic heterocycle.

## Description

### TECHNICAL FIELD

The present invention relates to cephem derivatives or pharmaceutically acceptable salts thereof useful as antibacterial agents and represented by the formula I: 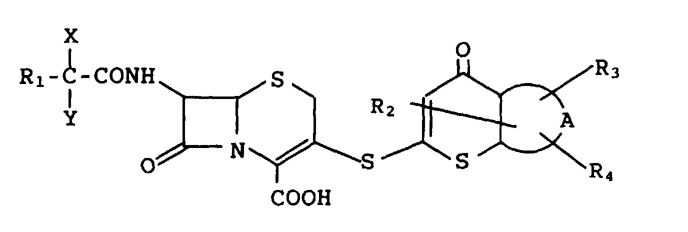 wherein 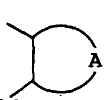 represents benzene ring, pyridine ring, pyrazine ring or 5-membered aromatic heterocycle (having one oxygen or sulfur atom as ring-constituting atom);
X and Y respectively represent hydrogen atom or CXY represents C=N-OH;
R₁ represents phenyl, thienyl or thiazolyl (which may be substituted with amino group or halogen atom),
R₂, R₃ and R₄ respectively represent hydrogen atom, halogen, hydroxy C₁-C₆ alkyl, isothiuronium C₁-C₆ alkyl, amino C₁-C₆ alkyl or amino C₁-C₆ alkylthiomethyl, there being no R₄ where 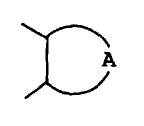 represents 5-membered aromatic heterocycle;
   or synthetic intermediates of the compounds of the formula I and represented by the formula II 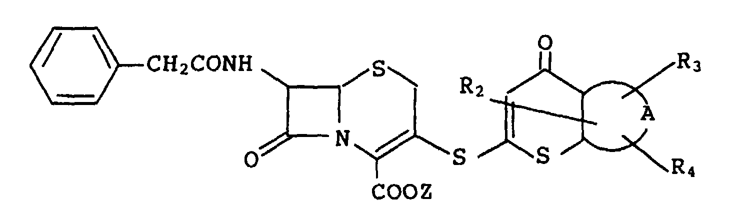 wherein Z represents a protective group for a carboxyl group and 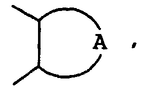 R₂, R₃ and R₄ are as defined above.

### BACKGROUND ART

In recent years, intractable infections due to pathogenic bacteria having resistance against antibiotics such as methicillin-resistant Staphylococcus aureus (MRSA) and vancomycin-resistant enterococci (VRE) cause serious problems in medical sites. Vancomycin, which was the only agent against which no resistant bacteria had been found, was frequently used as medicine against MRSA; however, recently, vancomycin-resistant Staphylococcus aureus (VRSA, Mu50) and heterovancomycin-resistant Staphylococcus aureus (hetero VRSA, Mu3) were found.

Among the above-mentioned resistant bacteria, MRSA are typical as hospital infecting bacteria and there are MRSA-carriers in healthy humans. Usually, these healthy MRSA-carriers are not sick with MRSA; however, they may tend to become sick with MRSA when they have come into compromised hosts with reduced vital resistance due to operations or other diseases. Once they become sick, remedy for their sickness is much hard to carry out. Nowadays, arbekacin, which is an aminoglycoside, and vancomycin, which is a glycopeptide, are used for the diseases. However, already, there are resistant bacteria against arbekacin; and resistant bacteria such as the above-mentioned Mu50 and Mu3 have been found also as to vancomycin only against which there were no resistant bacteria. Infection of Mu3 is found in all over Japan so that there is a fear that, in near feature, MRSA infectious diseses will increase which cannot be treated with vancomycin.

VRE provide world-shaking issues just as MRSA and have been found in Japan. VRE are bacteria which have resistance against various antibacaterial agents just as MRSA and which derive from enterococci (E. faecalis and E. faecium) against which in turn only vancomycin was effective and which have gained high resistance against vancomycin, too. It is highly feared that, in future, infection of VRE will be increased in Japan. However, no effective medicines are present now and have been developed yet.

Coming of such medical crysis is readily predicted and urgent development of medicines effective for the resistant bacteria has been desired to avert such crysis.

Cephem-type antibiotics have been proposed as new antibiotics effective for MRSA. More specifically, cephem derivatives having cyclic ammoniothiovinyl group at the 3-position have been proposed (Japanese Patent Provisional publication (Kokai) Nos. 6-206886 and 7-304779). Either of them is defective on antibacterial force and on toxicity probably deriving from the cyclic ammoniothiovinyl group, thus failing to develop for production of medicines.

Cephem derivatives having benzothiopyranylthiomethyl group at the 3-position have been proposed (Japanese Patent Provisional publication (Kokai) No. 5-32671). Though effectiveness against MRSA is disclosed in the Provisional publication, anti-bacterial activities of them is weak and extremely insufficient from the viewpoint of clinical applications. No disclosure is seen on effectiveness against VRE at all.

### DISCLOSURE OF THE INVENTION

We, the inventors, who made devoted researches to pursue cephem derivatives effective for MESA and VRE and having no defective ammonio group as mentioned above in structure, found cephem derivatives of the formula I having at the 3-position thiopyranylthio group with fused aromatic ring and having excellent antibacterial activities against MRSA and vancomycin-resistant E. faecalis, thus accomplishing the present invention.

The compounds and synthetic intermediates of the present invention are respectively represented by the formula I and II. The terms used for definition of letters in these formulae will be defined and exemplified in the following.

The term "C₁-C₆" refers to a group having 1 to 6 carbon atoms.

The "C₁-C₆ alkyl group" refers to a straight- or branched-chain alkyl group such as methyl, ethyl, n-propyl, isopropyl, n-butyl, tert-butyl, n-pentyl or n-hexyl.

The "amino C₃-C₇ alkyl group" refers to the above-mentioned "C₁-C₆ alkyl group" with amino group coupled to any of the carbon atoms.

The "isothiuronium C₁-C₆ alkyl group" refers to the above-mentioned "C₁-C₆ alkyl group" with isothiuronium group coupled to any of the carbon atoms.

The "hydroxy C₁-C₆ alkyl group" refers to the above-mentioned "C₁-C₆ alkyl group" with hydroxy group coupled to any of the carbon atoms.

The "halogen atom" may be fluorine, chlorine, bromine or iodine atom.

The "5-membered aromatic heterocycle" may be aromatic 5-membered heterocycle having one oxygen or sulfur atom as ring-constituting atom other than carbon atoms such as furan or thiophene.

When 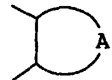 is 5-membered aromatic heterocycle, the thiopyranyl group with fused ring may be a group with any of the following structures. 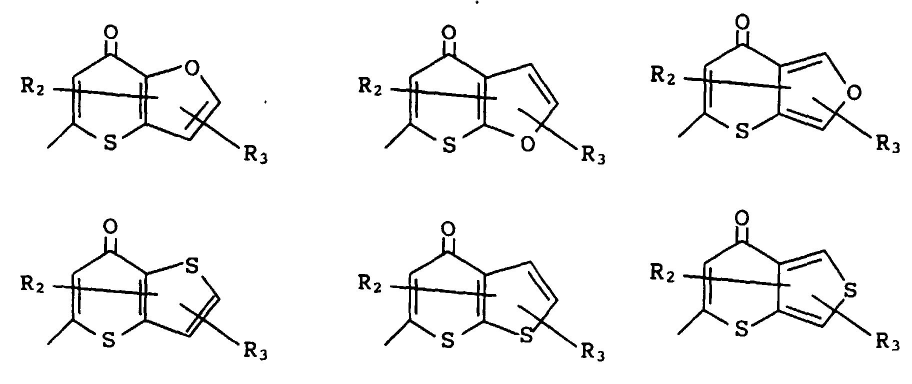 wherein R₂ and R₃ are as defined above.

The "protective group for the carboxyl group" may be a group which may be ordinarily utilized in the art and which may be readily removed. For example, it may be tri C₁-C₆ alkylsilyl such as trimethylsilyl, benzhydryl, p-methoxybenzyl, tert-butyl or p-nitrobenzyl.

The compounds according to the present invention may be as follows, though the present invention is not limited to these compounds.
- 7-Phenylacetamido-3-(7-oxo-7H-thieno[3,2-b]thiopyran-5-yl)thio-3-cephem-4-carboxylic acid
- 7-Phenylacetamido-3-(6-bromo-7-oxo-7H-thieno[3,2-b]thiopyran-5-yl)thio-3-cephem-4-carboxylic acid
- 7-Phenylacetamido-3-(3-bromo-7-oxo-7H-thieno[3,2-b]thiopyran-5-yl)thio-3-cephem-4-carboxylic acid
- 7-Phenylacetamido-3-(6-hydroxymethyl-7-oxo-7H-thieno[3,2-b]thiopyran-5-yl)thio-3-cephem-4-carboxylic acid
- 7-Phenylacetamido-3-(4-oxo-4H-1-benzothiopyran-2-yl)thio-3-cephem-4-carboxylic acid
- 7-Phenylacetamido-3-(5-aminomethyl-2-chloro-4-oxo-4H-thieno[2,3-b]thiopyran-6-yl)thio-3-cephem-4-carboxylic acid
- 7-Phenylacetamido-3-(7-oxo-7H-1-thiopyrano[3,2-b]furan-5-yl)thio-3-cephem-4-carboxylic acid
- 7-Phenylacetamido-3-(2-chloro-4-oxo-4H-thieno[2,3-b]thiopyran-6-yl)thio-3-cephem-4-carboxylic acid
- 7-Phenylacetamido-3-(2-bromo-4-oxo-4H-thieno[2,3-b]thiopyran-6-yl)thio-3-cephem-4-carboxylic acid
- 7-Phenylacetamido-3-(4-oxo-4H-thiopyrano[3,2-c]pyridin-2-yl)thio-3-cephem-4-carboxylic acid
- 7-Phenylacetamido-3-(4-oxo-4H-thiopyrano[3,2-b]pyridin-2-yl)thio-3-cephem-4-carboxylic acid
- 7-Phenylacetamido-3-(7-isothiuroniummethyl-4-oxo-4H-1-benzothiopyran-2-yl)thio-3-cephem-4-carboxylic acid
- 7-[2-(2-Thienyl)acetamido]-3-(7-oxo-7H-thieno[3,2-b]thiopyran-5-yl)thio-3-cephem-4-carboxylic acid
- 7-[2-Hydroxyimino-2-(2-aminothiazol-4-yl)acetamido]-3-(7-oxo-7H-thieno[3,2-b]thiopyran-5-yl)thio-3-cephem-4-carboxylic acid
- 7-[2-Hydroxyimino-2-(2-amino-5-chlorothiazol-4-yl)acetamido]-3-(6-hydroxymethyl-7-oxo-7H-thieno[3,2-b]thiopyran-5-yl)thio-3-cephem-4-carboxylic acid
- 7-[2-Hydroxyimino-2-(2-amino-5-chlorothiazol-4-yl)acetamido]-3-(6-chloro-7-oxo-7H-thieno[3,2-b]thiopyran-5-yl)thio-3-cephem-4-carboxylic acid
- 7-[2-Hydroxyimino-2-(2-amino-5-chlorothiazol-4-yl)acetamido]-3-[6-(2-aminoethylthiomethyl)-7-oxo-7H-thieno[3,2-b]thiopyran-5-yl]thio-3-cephem-4-carboxylic acid
- 7-[2-Hydroxyimino-2-(2-amino-5-chlorothiazol-4-yl)acetamido]-3-(8-oxo-8H-thiopyrano[2,3-b]pyrazin-6-yl)thio-3-cephem-4-carboxylic acid
- 7-[2-Hydroxyimino-2-(2-amino-5-chlorothiazol-4-yl)acetamido]-3-(7-oxo-7H-thieno[3,2-b]thiopyran-5-yl)thio-3-cephem-4-carboxylic acid
- 7-[2-Hydroxyimino-2-(2-amino-5-chlorothiazol-4-yl)acetamido]-3-(6-bromo-7-oxo-7H-thieno[3,2-b]thiopyran-5-yl)thio-3-cephem-4-carboxylic acid
- 7-[2-(2-Thienyl)acetamido]-3-(4-oxo-4H-1-benzothiopyran-2-yl)thio-3-cephem-4-carboxylic acid
- 7-[2-(2-Thienyl)acetamido]-3-(7-fluoro-4-oxo-4H-1-benzothiopyran-2-yl)thio-3-cephem-4-carboxylic acid
- 7-[2-(2-Thienyl)acetamido]-3-(7-chloro-4-oxo-4H-1-benzothiopyran-2-yl)thio-3-cephem-4-carboxylic acid
- 7-[2-(2-Thienyl)acetamido]-3-(7-hydroxymethyl-4-oxo-4H-1-benzothiopyran-2-yl)thio-3-cephem-4-carboxylic acid
- 7-[2-(4-Thiazolyl)acetamido]-3-(4-oxo-4H-1-benzothiopyran-2-yl)thio-3-cephem-4-carboxylic acid
- 7-[2-(2-Aminothiazol-4-yl)acetamido]-3-(4-oxo-4H-1-benzothiopyran-2-yl)thio-3-cephem-4-carboxylic acid
- 7-[2-Hydroxyimino-2-(2-aminothiazol-4-yl)acetamido]-3-(7-oxo-7H-thiopyrano[3,2-b]furan-5-yl)thio-3-cephem-4-carboxylic acid
- 7-[2-Hydroxyimino-2-(2-amino-5-chlorothiazol-4-yl)acetamido]-3-(6-chloro-7-oxo-7H-1-thiopyrano[3,2-b]furan-5-yl)thio-3-cephem-4-carboxylic acid
- 7-[2-Hydroxyimino-2-(2-amino-5-chlorothiazol-4-yl)acetamido]-3-(7-oxo-7H-1-thiopyrano[3,2-b]furan-5-yl)thio-3-cephem-4-carboxylic acid
- 7-[2-Hydroxyimino-2-(2-aminothiazol-4-yl)acetamido]-3-(7-fluoro-4-oxo-4H-1-benzothiopyran-2-yl)thio-3-cephem-4-carboxylic acid
- 7-[2-Hydroxyimino-2-(2-amino-5-chlorothiazol-4-yl)acetamido]-3-(4-oxo-4H-1-benzothiopyran-2-yl)thio-3-cephem-4-carboxylic acid
- 7-[2-(2-Thienyl)acetamido]-3-(7-oxo-7H-thiopyrano[3,2-b]furan-5-yl)thio-3-cephem-4-carboxylic acid
- 7-[2-(2-Thienyl)acetamido]-3-(7-oxo-7H-thieno[2,3-b]thiopyran-5-yl)thio-3-cephem-4-carboxylic acid
- 7-[2-(2-Thienyl)acetamido]-3-(2-chloro-4-oxo-4H-thieno[2,3-b]thiopyran-6-yl)thio-3-cephem-4-carboxylic acid
- 7-[2-Hydroxyimino-2-(2-aminothiazol-4-yl)acetamido]-3-(2-chloro-4-oxo-4H-thieno[2,3-b]thiopyran-6-yl)thio-3-cephem-4-carboxylic acid
- 7-[2-Hydroxyimino-2-(2-amino-5-chlorothiazol-4-yl)acetamido]-3-(6-hydroxymethyl-7-oxo-7H-thieno[3,2-b]thiopyran-5-yl)thio-3-cephem-4-carboxylic acid
- 7-[2-Hydroxyimino-2-(2-amino-5-chlorothiazol-4-yl)acetamido]-3-(2-chloro-4-oxo-4H-1-thieno[2,3-b]thiopyran-6-yl)thio-3-cephem-4-carboxylic acid

When CXY at the 7-position is C=N-OH (hydroxym), the following syn isomers (i) and anti isomers (ii) exist, the respective isomers and their mixtures being included in the compounds of the present invention. The syn isomers are preferable from the viewpoint of antibacterial force.

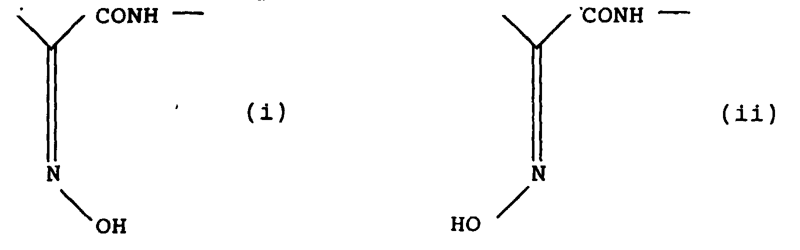

Moreover, the compounds of the invention may be in the form of pharmaceutically acceptable salts such as alkali salts, organic ammonium salts or acid addition salts. The appropriate alkali salts which can be used include, for example, potassium salt, sodium salt, calcium salt, magnesium salt, barium salt and ammonium salt. The appropriate acid addition salts which can be used include inorganic acid salts such as hydrochloride, hydrobromide, sulfate, nitrate and phosphate as well as organic acid salts such as acetate, oxalate, propionate, glycolate, lactate, pyruvate, malonate, succinate, maleate, fumarate, malate, tartrate, citrate, benzoate, cinnamate, methanesulfonate, benzenesulfonate, p-toluenesulfonate and salicylate.

The synthetic intermediates of the present invention are, as shown by the formula II, cephem compounds having carboxyl with protective group at the 4-position, phenylacetamido at the 7-position and substituent at the 3-position which is identical with that of the compounds I of the present invention.

The compounds of the present invention may be prepared by the following procedure.

### (1) Preparation of the synthetic intermediates of the present invention

The synthetic intermediates II of the present invention may be obtained by, as shown in process (1a) of the below-mentioned reaction scheme, reacting a cephem compound of the formula III with a mercaptothiopyran derivative of the formula IV in a solvent and, if needed, in the presence of tertiary amine such as ethyldiisopropylamine, triethylamine or pyridine.

There is no particular limitation on the solvent employed, providing that it is not involved with the reaction. For example, tetrahydrofuran (THF), dichloromethane, benzene, ethyl acetate, dimethylformamide (DMF), hexamethyl phosphoric triamide (HMPA), dimethyl sulfoxide (DMSO), acetone or mixture thereof may be used. Alternatively, the above-mentioned tertiary amine itself may be used as the solvent.

In the reaction, the aimed compound is obtained by reacting the 1-5 moles of the compound of the formula IV per mole of the compound of the formula III at the temperature range of ice cooling to room temperature for 1-6 hours.

The intermediates II according to the present invention may be obtained by, as shown in process 1b of the below-mentioned reaction scheme, reducing a cephem compund of the formula VI obtained in a procedure similar to that in the above-mentioned process (1a) using a cephem compound of the formula V and a mercaptothiopyran derivative of the formula IV.

The reduction process used may be a usual and conventional process in the field of cephem compound such as those in accordance with or substantially in accordance with the processes disclosed in J. Org. Chem. 54, 4962-4966 (1989), 59, 1606-1607 (1994), Pure Appl. Chem. 59, 1041-1046 (1987) and Japanese Patent Provisional Publication (Kokal) No. 10-36375.

More specifically, the aimed compounds are obtained by reacting the 1-3 moles of phosphorous trichloride or tribromide per mole of the compound of the formula VI in a solvent such as DMF, ethyl acetate or dichloromethane at the temperature range of -40 to 0°C for 1-60 minutes. 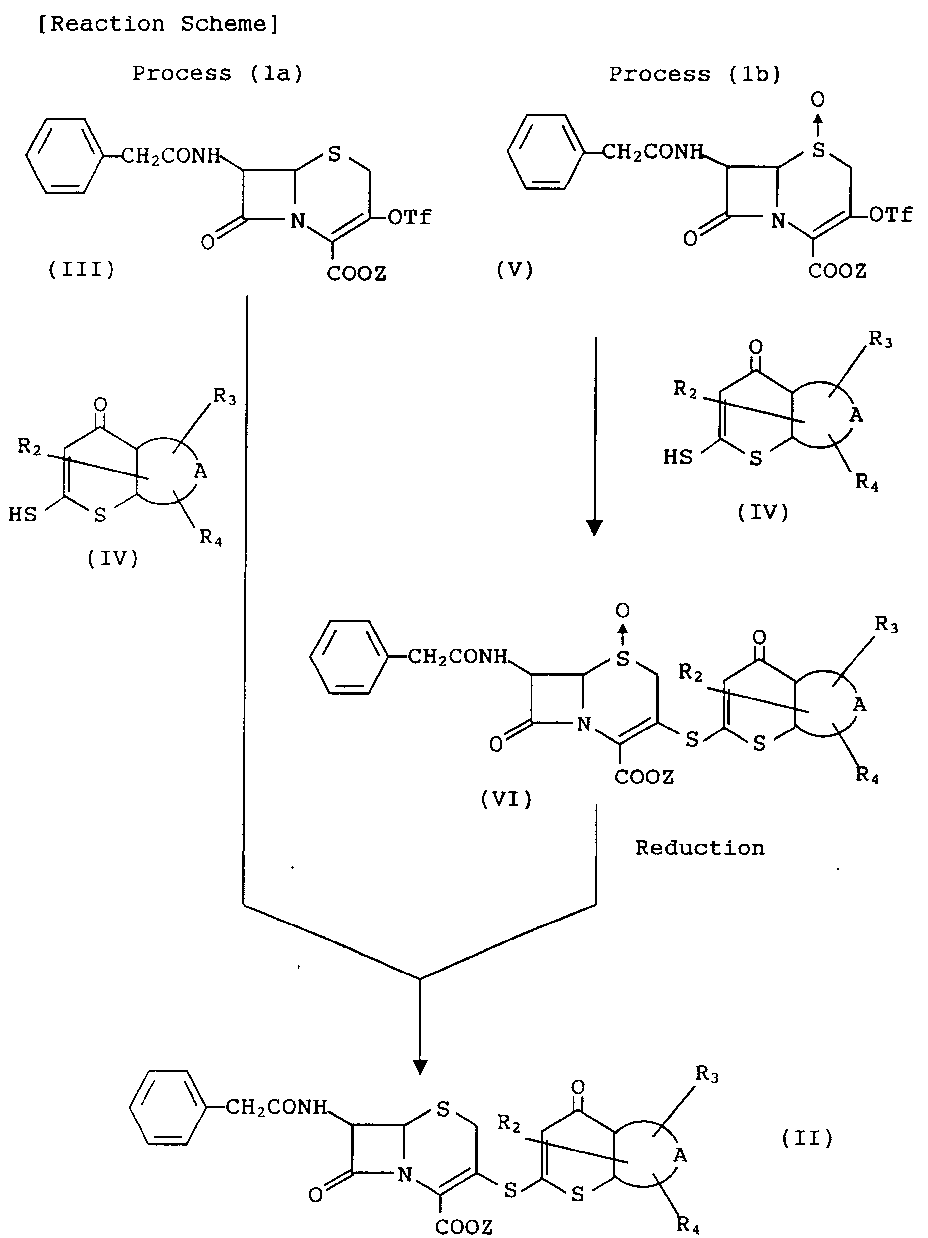 wherein Tf represents trifluoromethansulfonyl, 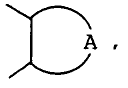 R₂, R₃, R₄ and Z are as defined above.

### (2) Preparation of the comounds of the present invention

The compounds I of the present invention are obtained by, as shown in process (2) of the below-mentioned reaction scheme, reacting a 7-amino compound, which is obtained by removing the phenylacetyl group at the 7-position from the synthetic intermediate of the formula II according to the present invention, with a compound of the formula VII in a solvent and removing the protective group for the carboxyl group at the 4-position from the resultant compound of the formula VIII in accordance with a usual manner.

Removal of the phenylacetyl group at the 7-position may be conducted by reacting the intermediate II with phosphorus pentachloride in accordance with the process disclosed in J. Antibiotics, 39, 394-403 (1986), using for example dichloromethane, chloroform or dimethylformamide as a solvent, in the presence of tertiary amine such as pyridine or triethylamine at the temperature range of -30 to 0°C for 0.5-3 hours.

Condensation reaction of the obtained 7-amino compound with the compound of the formula VII may be conducted in a solvent of for example dichloromethane, DMF or THF in the presence of dicyclocarbodiimide or N,N'-carbonyldiimidazole at the temperature rang of -20 to 40°C for 1-24 hours.

Removal of protective group for the carboxyl group at the 4-position may be conducted in the presence of hydrochloric acid, aluminium chloride, formic acid, trifluoroacetic acid or p-toluenesulfonic acid. There is no particular limitation on the solvent employed, providing that it is not involved with the reaction. For example, THF, dichloromethane, chloroform, benzene, ethyl acetate, DMF, acetone or mixture thereof may be used. The aimed compound is obtained by reacting 1-200 moles of the above-mentioned acid per mole of the compound of the formula VIII at the temperature range of ice cooling to room temperature for 1-6 hours. When trifluoroacetic acid is to be used for removal of the protective group, it is preferably reacted in the presence of for example anisole, thioanisole or phenol so as to faciliate the reaction and suppress any side reactions.

In the case where R₁ is phenyl and X and Y are respectively hydrogen atoms in the formula I according to the present invention, the aimed compound is obtained by removing the protective group for the carboxyl group at the 4-position directly from the synthetic intermediate II without conversion of cephem at the 7-position into the amino group. The reaction conditions in this case are the same as those in the above-mentioned process (2). 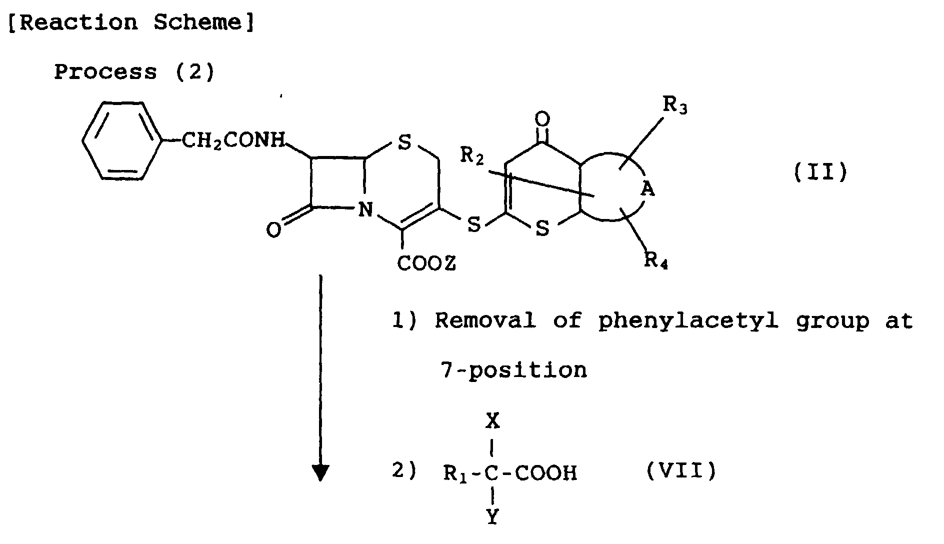 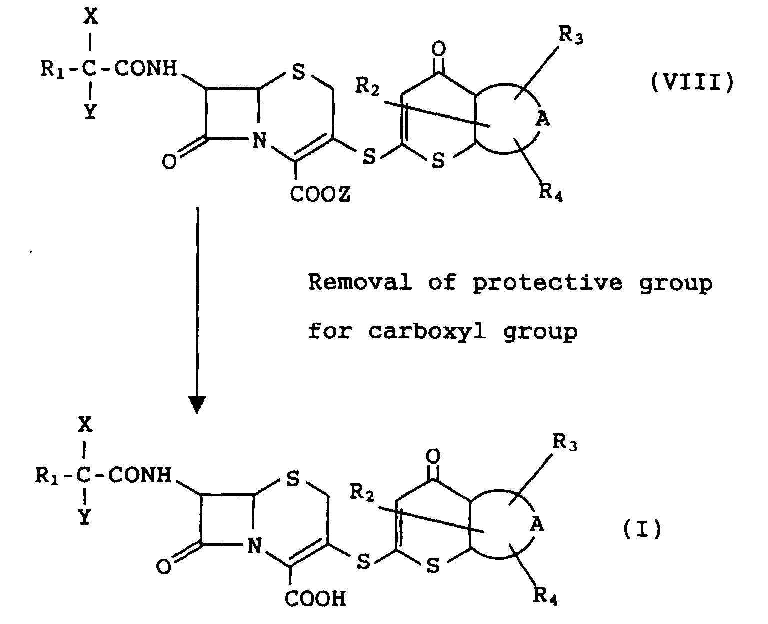 wherein 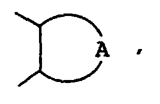 R₁, R₂, R₃, R₄, X, Y and Z are as defined above.

Thus obtained compounds of the present invention may be separated and purified as needs demand, according to an ordinary method such as extraction, condensation, neutralization, filtration, recrystallization or column chromatography.

Pharamaceutically acceptable salts of the compounds of the present invention may be prepared by various methods known in the art concerned.

Cephem compounds of the formulae III and V which are the starting materials in the above-mentioned processes are known compounds. Mercaptothiopyran derivatives of the formula IV which involve novel materials can be readily prepared substantially in accordance with the processes disclosed in Aust. J. Chem. 40, 1179-1190 (1987), Tetrahedron 35, 551-556 (1979), Z. Chem. 30, 245-246 (1990), Japanese Patent Provisional Publication (Kokai) No. 8-73304 or EP 481441.

Next, antibacterial activity of the compounds of the formula I according to the present invention will be described. The numbers of test compounds in this antibacterial test correspond to those in Examples referred to hereinafter.

Antibacterial test was performed in substantial accordance with agar plate dilution method based on Standard method of The Japan Society of Chemotherapy to determine minimum inhibitory concentration (MIC) of the respective test compounds against Staphylococcus aureus (S. aureus FDA 209P) and vancomycin-resistant enterococci (E. faecalis NCTC-12201). Also determined was MIC₈₀ on clinically separated strains (27 strains) of MRSA. The inoculated amount was 10⁶CFU/ml and flomoxef (FMOX) and vancomycin (VCM) were used as controls. The results are as shown in Table 1.

**Table 1**

| tested compound | MIC (µg/ml) | | |
|---|---|---|---|
| | S. aureus FDA 209P | E. faecalis NCTC-12201 | MRSA MIC₈₀ |
| compound 1 | 0.025 | 3.13 | 1.56 |
| compound 2 | 0.10 | 12.5 | 3.13 |
| compound 3 | 0.05 | 3.13 | 3.13 |
| compound 4 | 0.10 | 12.5 | 6.25 |
| compound 5 | 0.20 | 12.5 | 6.25 |
| compound 6 | 0.05 | 6.25 | 6.25 |
| compound 7 | ≦0.006 | 3.13 | 3.13 |
| compound 9 | 0.10 | 12.5 | 6.25 |
| compound 10 | 0.025 | 12.5 | 6.25 |
| compound 11 | 0.025 | 3.13 | 1.56 |
| compound 13 | 0.10 | 1.56 | 0.78 |
| compound 15 | 0.20 | 6.25 | 1.56 |
| compound 16 | 1.56 | 6.25 | 6.25 |
| compound 17 | 0.39 | 12.5 | 3.13 |
| compound 19 | 0.20 | 3.13 | 0.78 |
| compound 20 | 0.20 | 1.56 | 0.78 |
| compound 21 | 0.20 | 3.13 | 1.56 |
| compound 23 | 0.024 | 3.13 | 1.56 |
| compound 24 | 0.10 | 3.13 | 0.78 |
| FMOX | 0.20 | 100 | 100 |
| VCM | 0.78 | >1000 | 1.56 |

As is clear from the above, the compounds of the present invention of the formula I are effective for MRSA and vancomycin-resistant E. faecalis and can be applied for treatment of infections caused by MRSA or other pathogenic bacteria.

The compounds of the present invention may be administered to human or mammal orally or parenterally. In oral administration, the compounds may be in the form of tablets, coated tablets, powders, granules, capsules, microcapsules, syrups and the like; and in parenteral administration, in the form of injections which may include soluble freeze-drying form, suppositories and the like. In the preparation of these forms, pharmaceutically acceptable excipient, binders, lubricants, disintegrators, suspensions, emulsifiers, antiseptics, isotonics, stabilizers and dispersing agents, for example, lactose, sucrose, starch, dextrin, crystalline cellulose, kaolin, calcium carbonate, talc, magnesium stearate, distilled water, physiological saline solution and amino acid infusion may be used.

The dosage for humans may depend on the condition of the disease to be treated, the age and weight of the patient and the like. A daily dosage for an adult may be in the range of from 100 to 5,000 mg and may be given in divided doses 1 to 4 times a day.

### BEST MODE FOR CARRYING OUT THE INVENTION

The present invention is more specifically illustrated with reference to the following preparations and examples. It is to be, however, noted that the present invention is not limited to these

### Preparation 1: Preparation of 5-mercapto-7-oxo-7H-thiopyrano[3,2-b]furan

(1) In accordance with the process disclosed in Tetrahedron, 35, 551 (1979), 2-acetyl-3-bromofuran (10.0 g, 53.2 mmol) and carbon disulfide (7.0 g. 93.2 mmol) were dissolved in anhydrous dimethylformamide (70 ml) and the solution was added with 55% sodium hydride (4.0 g, 93.2 mmol) over about 1.5 hours while it was stirred and inner temperature was maintained below 10°C under ice cooling. After further stirring for 0.5 hour at the temperature, the solution was added with methanol (1.1 ml, 26.6 mmol) and further stirred at room teperature for 40 minutes. Then, the solution was heated and stirred in the boiling water bath for 1 hour and allowed to cool to room temperature. Under water cooling, the solution was added dropwise with methyl iodide (22.6 g, 159.6 mmol) and stirred at room temperature for 24 hours. The solution was poured into ice water, extracted with dichloromethane (300 ml × 2 times), washed with water and dried over anhydrous magnesium sulfate. The solvent was removed under reduced pressure and the residue was recrystallized from ethanol using active carbon to obtain 5.9 g (yield: 56%) of 5-methylthio-7-oxo-7H-thiopyrano[3,2-b]furan as colorless needles.
   Melting Point: 149-150°C
   NMR(CDCl₃)δ: 2.63(3H, s), 6.74(1H, d, J=2.0Hz), 7.01(1H, s), 7.80(1H, d, J=2.0Hz)
(2) The obtained compound (5.7 g, 29 mmol) was dissolved in dichloromethane and added dropwise with dichloromethane solution (150 ml) of 80% metachloroperbenzoic acid (5.9 g, 27 mmol) under ice-water cooling and over about 45 minutes. After further stirring at the temperature for one hour, the precipitates were collected by filtration, washed with dichloromethane and dried over air. This solid was stirred for a while in the aqueous solution (100 ml) of diluted sodium hydrogencarbonate and collected by filtration, washed with water and acetone, successively, to obtain 3.3 g (yield: 53%) of 5-methylsulfinyl-7-oxo-7H-thiopyrano[3,2-b]furan as colorless powder.
   Melting Point: 230-232°C
   NMR(DMSO-d₆)δ: 2.99(3H, s), 7.35(1H, d, J=2.0Hz), 7.46(1H, s), 8.40(1H, d, J=2.0Hz)
(3) The obtained compound (3.3 g, 15 mmol) was dissolved in tetrahydrofuran (160 ml), added with about 70% sodium hydrogensulfide (10 g) and stirred at room temperature for 24 hours. The solvent was removed under reduced pressure, added with water (150 ml) for dissolution and washed with dichloromethane. The water layer was acidified with 2N hydrochloric acid under cooling into pH 1-2. The precipitates were collected by filtration and washed with water and ether, successively, to obtain 2.3 g (yield: 82%) of the titled compound as yellow-red powder.
   Melting Point: >245°C
   NMR(DMSO-d₆)δ: 7.05 and 7.07(2H, m), 8.31(1H, d, J=1.6Hz)

The following compounds were obtained from the corresponding starting materials and in a process similar to that in Preparation 1.
- 5-Mercapto-7-oxo-7H-thieno[3,2-b]thiopyran
   Melting Point: 151-153°C
   NMR(DMSO-d₆)δ: 7.03(1H, s), 7.45(1H, d, J=5.3Hz), 8.24(1H, d, J=5.3Hz)
- 2-Chloro-6-mercapto-4-oxo-4H-thieno[2,3-b]thiopyran
   Melting Point: >245°C
   NMR(DMSO-d₆)δ: 7.00(1H, s), 7.51(1H, s)
- 2-Mercapto-4-oxo-4H-thiopyrano[3,2-c]pyridine
   Melting Point: >200°C (decomp.)
   MS m/z: 195 (M⁺)
   NMR(CDCl₃)δ: 6.95(1H, s), 7.75(1H, d, J=5.9), 8.62(1H, d, J=5.9Hz), 9.22(1H, s)
- 2-Mercapto-4-oxo-4H-thiopyrano[3,2-b]pyridine
   Melting Point: >200°C (decomp.)
   MS m/z: 195 (M⁺)
   NMR(DMSO-d₆)δ: 7.16(1H, s), 7.74(1H, dd, J=4.6Hz, 8.2Hz), 8.20 (1H, dd, J=1.3Hz, 8.2Hz), 8.77(1H, dd, J=1.3Hz, 4.6Hz)
- 6-Mercapto-8-oxo-8H-thiopyrano[2,3-b]pyrazine
   Melting Point: >178°C (decomp.)
   NMR(DMSO-d₆)δ: 7.31(1H, br), 8.81(2H, br)
- 3-Bromo-5-mercapto-7-oxo-7H-thieno[3,2-b]thiopyran
   Melting Point: 199-202°C (decomp.)
   NMR(DMSO-d₆)δ: 6.99(1H, s), 8.41(1H, s)
- 2-Bromo-6-mercapto-4-oxo-4H-thieno[2,3-b]thiopyran
   Melting Point: 100-103°C (decomp.)
   NMR(DMSO-d₆)δ: 6.97(1H, s), 7.65(1H, s)

### Preparation 2: Preparation of 5-tert-butoxycarbonylaminomethyl-2-chloro-6-mercapto-4-oxo-4H-thieno[2,3-b]thiopyran

(1) 2-Chloro-6-methylthio-4-oxo-4H-thieno[2,3-b]thiopyran (6.64 g, 0.027 mol), diethylamine hydrochloride (19.65 g, 0.24 mol) and paraformaldehyde (17.16 g) were added to 80% aqueous solution of acetic acid and refluxed under heating for 30 hours. The solvent was removed under reduced pressure. The residue was added with ice water and stirred for a while, and supernatant was removed. The remaining viscous matter was dissolved in dimethylformamide (150 ml), filtered for removal of the insoluble matter. Then, the filtrate was added with water (40 ml), methanol (100 ml) and potassium carbonate (37 g, 0.7 mol) and stirred for 35 minutes. Then, ice water (700 ml) was added and the resulting precipitates were collected by filtration. The precipitates were recrystallized from ethanol to obtain 3.5 g (yield: 46.5%) of 2-chloro-5-hydroxymethyl-6-methylthio-4-oxo-4H-thieno[2,3-b]thiopyran.
   MS m/z: 278(M⁺)
(2) The obtained compound (2 g, 7.17 mmol) was dissolved in dichloromethane (30 ml), added with triethylamine (2 ml, 14.34 mmol) under ice cooling, and added dropwise with methanesulfonyl chloride (0.89 ml, 11.47 mmol). The cooling bath was removed and the reaction mixture was stirred at room temperature for 25 hours and added with dichloromethane (150 ml). The reaction mixture was washed with water (50 ml) and dried over anhydrous magnesium sulfate. The solvent was removed under reduced pressure and the residue was washed with ether to obtain 2 g (yield: 94%) of 2-chloro-5-chloromethyl-6-methylthio-4-oxo-4H-thieno[2,3-b]thiopyran.
   MS m/z: 296(M⁺)
   NMR(CDCl₃)δ: 2.69(3H, s), 4.88(2H, s), 7.54(1H, s)
(3) The obtained compound (2 g, 6.73 mmol) was suspended in dimethylformamide (40 ml), added with sodium azide (1.31 g, 20.19 mmol) while stirred at room temperature, and further stirred for 3 hours. Ice water was added and the resulting precipitates were collected by filtration and washed with water to obtain 1.9 g (yield: 93%) of 5-azidomethyl-2-chloro-6-methylthio-4-oxo-4H-thieno[2,3-b]thiopyran.
   MS m/z: 303(M⁺)
   IR (KBr): 2093 (-N₃) cm⁻¹
   NMR (CDCl₃)δ: 2.66(3H, s), 4.62(2H, s), 7.55(1H, s)
(4) The obtained compound (1.48 g, 4.87 mmol) was dissolved in tetrahydrofuran (40 ml), added with triphenylphosphine (2 g, 7.78 mmol) and water (0.4 ml), successively, and stirred at room temperature for 24 hours. Then, the reaction mixture was added with di-tert-butyldicarbonate (1.7 g, 7.79 mmol) and further stirred at room temperature for 24 hours. Then, the reaction mixture was added with dichloromethane (150 ml), washed with water and dried over anhydrous magnesium sulfate. The solvent was removed under reduced pressure and the residue was purified by silica gel column chromatography (Wako Gel™ C-200 : dichloromethane-ethyl acetate = 8 : 1) to obtain 1.5 g (yield: 82%) of 5-tert-butoxycarbonylaminomethyl-2-chloro-6-methylthio-4-oxo-4H-thieno[2,3-b]thiopyran.
   MS m/z: 377(M⁺)
(5) The obtained compound (500 mg, 1.32 mmol) and 80% metachloroperbenzoic acid (299 mg, 1.39 mmol) were used to conduct the procedure similar to that shown in (2) of Preparation 1 to obtain 450 mg (yield: 87%) of 5-tert-butoxycarbonyl-aminomethyl-2-chloro-6-methylsulfinyl-4-oxo-4H-thieno[2,3-b]thiopyran. Then, this compound (400 mg, 1.02 mmol) and sodium hydrogensulfide (163 mg, 2.1 mmol) were used to conduct the procedure similar to that shown in (3) of Preparation 1 to obtain 320 mg (yield: 95%) of the titled compound as yellow powder.
   Melting Point: 128-131°C (decomp.)
   NMR (DMSO-d₆)δ: 1.40(9H, s), 4.48(2H, s), 7.53(1H, s)

### Preparation 3: Preparation of sodium 5-mercapto-7-oxo-6-(2-tetrahydropyranyl)oxymethyl-7H-thieno[3,2-b]thiopyran

(1) 5-Methylthio-7-oxo-7H-thieno-[3,2-b]thiopyran (1.77 g, 8.24 mmol) and dimethylamine hydrochloride (6.05 g, 74.2 mmol), paraformaldehyde (5.29 g) and potassium carbonate (2.28 g) were used to conduct the procedure similar to that shown in (1) of Preparation 2 to obtain 1.54g (yield: 77%) of 6-hydroxymethyl-5-methylthio-7-oxo-7H-thieno[3,2-b]thiopyran.
   NMR (CDCl₃)δ: 2.66(3H, s), 3.76(1H, t, J=7.0Hz), 4.94(2H, d, J=7.0Hz), 7.25(1H, d, J=5.3Hz), 7.85(1H, d, J=5.3Hz)
(2) The obtained compound (2.16 g, 8.84 mmol) was suspended in dichloromethane (120 ml), added with 3,4-dihydro-2H-pyran (8 ml, 87.68 mmol) and p-toluenesulfonic acid monohydrate (32.8 mg, 0.172 mmol), successively, under ice-cooled stirring and stirred at room temperature for 4 hours. Then, the reaction mixture was added with dichloromethane (100 ml), washed with an aqueous solution of saturated sodium hydrogencarbonate (50 ml × 2 times) and saturated saline solution, successively, and dried over anhydrous magnesium sulfate. The solvent was removed under reduced pressure and the residue was purified by column chromatography (Wako Gel™ C-200 : dichloromethane-ethyl acetate = 8 : 1) to obtain 2.26 g (yield: 78%) of 5-methylthio-7-oxo-6-(2-tetrahydropyranyl)oxymethyl-7H-thieno[3,2-b]thiopyran.
   NMR(CDCl₃)δ: 1.51-1.84(6H, m), 2.66(3H, s), 3.58-3.64(1H, m), 3.98-4.09(1H, m), 4.74(1H, d, J=10.6Hz), 4.87-4.89(1H, m), 5.05(1H, d, J=10.6Hz), 7.21(1H, d, J=5.3Hz), 7.76(1H, d, J=5.3Hz)
(3) The obtained compound (1.26 g, 3.83 mmol) and metachloroperbenzoic acid (660 mg, 3.82 mmol) were used to conduct the procedure similar to that shown in (2) of Preparation 1 to obtain 839 mg (yield: 64%) of 5-methylsulfinyl-7-oxo-6-(2-tetrahydropyranyl)oxymethyl-7H-thieno[3,2-b]thiopyran.
   NMR(CDCl₃)δ: 1.56-1.84(6H, m), 3.07(3H, d, J=7.0Hz), 3.56-3.66(1H, m), 3.87-3.98(1H, m), 4.46(1H, d, J=12.2Hz), 4.74(1H, d, J=12.2Hz), 4.81-4.84(1H, m), 4.99(1H, d, J=12.2Hz), 5.31(1H, d, J=12.2Hz), 7.39(1H, d, J=5.3Hz), 7.89(1H, d, J=5.3Hz)
(4) The obtained compound (482 mg, 1.4 mmol) was dissolved in tetrahydrofuran (15 ml) and added with 1N sodium hydrogensulfide (2.1 ml) under ice-water cooling, and stirred at room temperature for 30 minutes. The solvent was removed under reduced pressure to obtain the titled compound. This compound is used in the succeeding reaction without further purification.

### Preparation 4: Preparation of sodium 6-bromo-5-mercapto-7-oxo-7H-thieno[3,2-b]thiopyran

(1) 5-Methylthio-7-oxo-7H-thieno[3,2-b]thiopyran (2.14 g, 10 mmol) was dissolved in acetic acid (140 ml) and added dropwise with bromine (0.96 ml). The reaction mixture was stirred at 50°C for 16 hours. The resulting precipitates were collected by filtration, washed with water and dried. The reaction product was purified by column chromatography(Wako Gel™ C-200: dichloromethane-ethyl acetate = 15 : 1) to obtain 1.98 g (yield: 67%) of 6-bromo-5-methylthio-7-oxo-7H-thieno[3,2-b]thiopyran.
   NMR(CDCl₃)δ: 2.68(3H, s), 7.26(1H, d, J=5.3Hz), 7.79(1H, d, J=5.3Hz).
(2) The obtained compound (2.77 g, 9.4 mmol) and metachloroperbenzoic acid (1.96 g, 9.1 mmol) were used to conduct the procedure similar to that shown in (2) of Preparation 1 to obtain 2.31 g (yield: 79%) of 6-bromo-5-methylsulfinyl-7-oxo-7H-thieno[3,2-b]thiopyran.
   NMR(CDCl₃)δ: 3.07(1H, s), 7.44(1H, d, J=5.3Hz), 7.92(1H, d, J=5.3Hz)
(3) The obtained compound (430 mg, 1.4 mmol) and 1N sodium hydrogensulfide (2.1 ml) were used to conduct the procedure similar to (4) in Preparation 3 to obtain the titled compound.

### Preparation 5: Preparation of sodium 6-chloro-5-mercapto-7-oxo-7H-thieno[3,2-b]thiopyran

(1) 5-Methylthio-7-oxo-7H-thieno[3,2-b]thiopyran (128 mg, 0.6 mmol) dissolved in N,N-dimethylformamide (2 ml) was added with N-chlorosuccinimide (120.2 mg, 0.9 mmol) and stirred at room temperature for 2 hours. The reaction mixture was added with water (10 ml) and the resulting precipitate was filtered out. The resulting product was further fully washed with water to obtain 120 mg (yield: 80%) of 6-chloro-5-methylthio-7-oxo-7H-thieno[3,2-b]thiopyran as colorless powder.
   MS m/z: 248 (M⁺)
   NMR(CDCl₃)δ: 2.66(3H, s), 7.25(1H, d, J=5.0 Hz), 7.80(1H, d, J=5.0 Hz)
(2) The obtained compound (560 mg, 2.25 mmol) and 80% metachloroperbenzoic acid (504.8 mg, 2.34 mmol) were used to conduct the procedure similar to that shown in (2) of Preparation 1 to obtaining 520 mg (yield: 87.4%) of 6-chloro-5-methylsulfinyl-7-oxo-7H-thieno[3,2-b]thiopyran as colorless powder.
   MS m/z: 264(M⁺)
   NMR(CDCl₃)δ: 3.05(3H, s), 7.44(1H, d, J=5.2 Hz), 7.94(1H, d, J=5.2 Hz)
(3) The obtained compound (600 mg, 2.27 mmol) and 1N sodium hydrogensulfide (3.5 ml, 3.5 mmol) were used to conduct the procedure similar to that shown in (4) of Preparation 3, thus obtaining tetrahydrofuran solution including 2.27 mmol of the titled compound.

### Preparation 6: Preparation of sodium 6-[2-(tert-butoxycarbonyl)aminoethylsulfinylmethyl]-5-mercapto-7-oxo-7H-thieno[3,2-b]thiopyran

(1) 6-Hydroxymethyl-5-methyltio-7-oxo-7H-thieno[3,2-b]thiopyran dissolved in dimethylformamide (20 ml) was added with thionyl chloride (1.98 g, 16.67 mmol) at the temperature range of 0-5°C and stirred for 2 hours. The resulting mixture was added with ice water (100 g) and the precipitating crystals were filtered, washed with water and dried under reduced pressure in a desiccator in the presence of diphosphorus pentoxide to obtain 1.55 g (yield: 88%) of 6-chloromethyl-5-methylthio-7-oxo-7H-thieno[3,2-b]thiopyran as yellow-red powder.
   NMR(CDCl₃)δ: 2.71(3H, s), 4.93(1H, s), 7.23(1H, d, J=5.3 Hz), 7.81(1H, d, J=5.3 Hz)
(2) The obtained compound (1.27 g, 4.83 mmol) was dissolved in dichloromethane (70 ml) and added with triethylamine (4.88 g, 48.30 mmol) and 2-[(tert-butoxycarbonyl)amino]-1-ethanethiol (2.57 g, 14.49 mmol), successively, and stirred at room temperature for 18 hours. The resulting mixture was washed with water (30 ml) and saturated saline solution (30 ml), successively, and dried over anhydrous magnesium sulfate. The solvent was removed under reduced pressure and the residue was purified by column chromatography (Wago Gel™ C-200, hexane―ethyl acetate-dichloromethane (4 : 3: 3)) to obtain 1.65 g (yield: 84%) of 6-[2-(tert-butoxycarbonyl)amino-ethylthiomethyl]-5-methylthio-7-oxo-7H-1-thieno[3,2-b]thiopyran as yellow oil.
   NMR(CDCl₃)δ: 1.43(9H, s), 2.68(3H, s), 2.77(2H, t, J=6.3 Hz), 3.43(2H, t, J=6.3 Hz), 4.06(2H, s), 7.22(1H, d, J=5.3 Hz), 7.88(1H, d, J=5.3 Hz)
(3) The obtained compound (1.05 g, 2.60 mmol) and 80% metachloroperbenzoic acid (1.29 g, 5.98 mmol) were used to conduct the procedure similar to that shown in (2) of Preparation 1, thus obtaining 741 mg (yield: 65%) of 6-[2-(tert-butoxycarbonyl)aminoethylsulfinylmethyl]-5-methylsulfinyl-7-oxo-7H-thieno[3,2-b]thiopyran (diastereomer mixture) as yellow froth.
   NMR(CDCl₃)δ: 1.42(4.5H, s), 1.45(4.5H, s), 2.54-2.64(0.5H, m), 2.91-3.20(1H, m), 3.23(1.5H, s), 3.24(1.5H, s), 3.27-3.32(0.5H, m), 3.48-3.54(1H, m), 3.70-3.75(1H, m), 4.08(0.5H, d, J=12.2Hz), 4.43(0.5H, d, J=13.3Hz), 4.50(0.5H, d, J=12.2Hz), 4.67(0.5H, d, J=13.3Hz), 7.43-7.45(1H, m), 7.95-7.98(1H, m)
(4) The obtained compound (878 mg, 2.0 mmol) and 1N sodium hydrogensulfide (2.0 ml, 2.0 mmol) were used to conduct the procedure similar to that shown in (4) of Preparation 3 to obtain tetrahydrofuran solution including 2.0 mmol of the titled compound.

### Preparation 7: Preparation of 7-hydroxy-2-mercapto-4-oxo-4H-1-benzothiopyran

(1) 7-Methoxy-2-mercapto-4-oxo-4H-1-benzothiopyran (2.00 g, 8.92 mmol) dissolved in DMF (15 ml) was gradually added with sodium hydride (505.8 mg, 11.59 mmol) over 30 minutes in the ice bath and under nitrogen atmosphere. After removal of the ice bath and stirring for 12 hours, the solution was added with methyl iodide (0.83 ml, 13.37 mmol) and further stirred for 3 hours. The reaction liquid was poured into the cooled aqueous solution (100 ml) of saturated ammonium chloride. The precipitated crude crystals were filtrated and fully washed with water and dried in a desiccator under reduced pressure. The crude crystals were washed with the ether-hexane solution to obtain 1.77 g (yield: 83%) of 7-methoxy-2-methylthio-4-oxo-4H-1-benzothiopyran as pale yellow powder.
   Melting Point: 133-135°C
   NMR(CDCl₃)δ: 2.61(3H, s), 3.89(3H, s), 6.82(1H, s), 6.91(1H, d, J=2.6Hz), 7.65(1H, dd, J=2.6Hz, 8.9Hz), 8.38(1H, d, J=8.9Hz)
(2) The obtained compound (570 mg, 2.39 mmol) was added with hydrobromic acid (20 ml) and refluxed in heating for 28 hours. After allowed to cool, the solution was neutralized with the aqueous solution of 20% sodium hydroxide under cooling in the ice bath. The precipitated crude crystals were filtrated and fully washed with water and dried in a desiccator under reduced pressure. The crude crystals were washed with ether to obtain 520 mg (yield: 97%) of 7-hydroxy-2-methylthio-4-oxo-4H-1-benzothiopyran as pale purple powder.
   Melting Point: 231-232°C
   NMR(DMSO-d₆)δ: 2.69(3H, s), 6.74(1H, s), 7.01(1H, d, J=2.3Hz), 7.05(1H, brs), 8.16(1H, d, J=8.6Hz), 10.77(1H, s)
(3) The obtained compound (540 mg, 2.40 mmol) and 80% metachloroperbenzoic acid (860 mg, 3.75 mmol) were used to conduct the procedure similar to that shown in (2) of Preparation 1 to obtain 559 mg (yield: 97%) of 7-hydroxy-2-methylsulfinyl-4-oxo-4H-1-benzothiopyran as yellow powder.
   NMR(DMSO-d₆)δ: 3.08(3H, s), 7.18(1H, dd, J=2.3Hz, 8.9Hz), 7.27(1H, d, J=2.3Hz), 7.33(1H, d, J=2.3Hz), 8.29(1H, d, J=8.9Hz), 11.02(1H, brs)
(4) The obtained compound (559 mg, 2.32 mmol) and sodium hydrogensulfide (1.05 g, 13.12 mmol) were used to conduct the procedure similar to that shown in (3) of Preparation 1 to obtain 324 mg (yield: 66%) of the titled compound as yellow powder.
   Melting Point: 160-163°C
   NMR(DMSO-d₆)δ: 7.01-7.07(3H, m), 8.11(1H, d, J=8.6Hz), 10.91(1H, br)

### Preparation 8: Preparation of sodium 6-methoxymethyloxymethyl-5-mercapto-7-oxo-7H-thieno[3,2-b]thiopyran

(1) 6-Hydroxymethyl-5-methylthio-7-oxo-7H-thieno[3,2-b]thiopyran (1.46 g, 5.97 mmol) obtained in (1) of the Preparation 3 and dissolved in dichloromethane (100 ml) was added with N-ethyldiisopropylamine (1.54 g, 11.94 mmol) and chloromethylmethylether (0.72 g, 8.95 mmol), successively, at the temperature range of 0-5°C and stirred at the same temperature for 1 hour. After further stirring at room temperature for 8 hours, the solvent was removed and the residue was added with ethyl acetate (250 ml), washed with water and saturated saline solution, successively, and dried over anhydrous magnesium sulfate. The solvent was removed and the residue was purified by column chromatography (Wago Gel™ C-200, hexane―ethyl acetate (1 : 2) to obtain 1.07 g (yield: 62%) of 6-methoxymethyloxymethyl-5-methylthio-7-oxo-7H-thieno[3,2-b]thiopyran.
   MS m/z: 288(M⁺)
   NMR(CDCl₃)δ: 2.67(3H, s), 3.46(3H, s), 4.77(2H, s), 4.88(2H, s), 7.21(1H, d, J=5.3Hz), 7.77(1H, d, J=5.3Hz)
(2) The obtained compound (1.00 g, 4.09 mmol) and 80% metachloroperbenzolc acid (882 mg, 4.09 mmol) were used to conduct the procedure similar to that shown in (2) of Preparation 1 to obtain 843 mg (yield: 67%) of 6-methoxymethyloxymethyl-5-methylsulfinyl-7-oxo-7H-thieno[3,2-b]thiopyran.
   MS m/z: 304(M⁺)
   NMR(CDCl₃)δ: 3.06(3H, s), 3.44(3H, s), 4.56(1H, d, J=11.9Hz), 4.71(1H, d, J=6.3Hz), 4.77(1H, d, J=6.3Hz), 5.09(1H, d, J=11.9Hz), 7.40(1H, d, J=5.3Hz), 7.90(1H, d, J=5.3Hz)
(3) The obtained compound (660 mg, 2.17 mmol) and 1N sodium hydrogensulfide (2.3 ml) were used to conduct the procedure similar to that shown in (4) of Preparation 3 to obtain tetrahydrofuran solution including 2.17 mmol of the titled compound.

### Preparation 9: Preparation of sodium 6-chloro-5-mercapto-7-oxo-7H-thiopyrano[3,2-b]furan

(1) 5-Methylthio-7-oxo-7H-thiopyrano[3,2-b]furan (500 mg, 2.52 mmol) and N-chlorosuccinimide (438 mg, 3.27 mmol) were used to conduct the procedure smilar to that shown in (1) of Preparation 5 to obtain 380 mg (yield: 65%) of 6-chloro-5-methylthio-7-oxo-7H-thiopyrano[3,2-b]furan.
   MS m/z: 232(M⁺)
   NMR(CDCl₃)δ: 2.68(3H, s), 6.80(1H, d, J=2.0Hz), 7.83(1H, d, J=2.0Hz)
(2) The obtained compound (376 mg, 1.61 mmol) and 80% metachloroperbenzoic acid (416 mg, 1.93 mmol) were used to conduct the procedure similar to that shown in (2) of Preparation 1 to obtain 239 mg (yield: 60%) of 6-chloro-5-methylsulfinyl-7-oxo-7H-thiopyrano[3,2-b]furan.
   MS m/z: 248(M⁺)
   NMR(CDCl₃)δ: 3.05(3H, s), 6.98(1H, d, J=2.0Hz), 7.94(1H, d, J=2.0Hz)
(3) The obtained compound (524 mg, 2.10 mmol) and 1N-sodium hydrogensulfide (2.5 ml) were used to conduct the procedure similar to that shown in (4) of Preparation 3 to obtain tetrahydrofuran solution including 2.10 mmol of the titled compound.

### Example 1: Preparation of 7-pheylacetamido-3-(7-oxo-7H-thieno[3,2-b]thiopyran-5-yl)thio-3-cephem-4-carboxylic acid (Compound 1)

(1) 7-Phenylacetamido-3-trifluoromethanesulfonyloxy-3-cephem-4-carboxylic acid benzhydryl ester (160 mg, 0.25 mmol) dissolved in dimetylformamide (10 ml) was added with 5-mercapto-7-oxo-7H-thieno[3,2-b]thiopyran (76 mg, 0.38 mmol) and N-ethyldiisopropylamine (22 mg, 0.17 mmol), successively, and stirred at room temperature for 2 hours. The reaction product was added with ethyl acetate (100 ml), washed with an aqueous solution of saturated sodium hydrogencarbonate (50 ml × 2 times) and dried over anhydrous magnesium sulfate. The solvent was removed under reduced pressure and the residue was purified by column chromatography (Wago Gel™ C-200, hexane―ethyl acetate (1 : 2)) to obtain 120 mg (yield: 70.2%) of 7-phenylacetamido-3-[(7-oxo-7H-thieno[3,2-b]thiopyran-5-yl)thio]-3-cephem-4-carboxylic acid benzhydryl ester as yellow froth.
   NMR(CDCl₃)δ: 3.42(1H, d, J=17.8Hz), 3.64(1H, d, J=17.8Hz), 3.65(2H, d, J=3.3Hz), 5.03(1H, d, J=5.3Hz), 5.91(1H, dd, J=5.0Hz, 9.2Hz), 6.37(1H, d, J=9.2Hz), 6.98(1H, s), 6.99(1H, s), 7.19-7.36(16H, m), 7.85(1H, d, J=5.3Hz)
(2) The obtained compound (118 mg, 0.17 mmol) was dissolved in dichloromethane (8 ml), added with anisole (380 mg, 3.51 mmol) and trifluoroacetic acid (400 mg, 3.1 mmol), successively, and stirred at room temperature for 4 hours. The reaction mixture was cooled to 0-5°C and added with isopropyl ether (20 ml) under stirring. The precipitating precipitates were collected by filteration and fully washed with isopropyl ether to obtain 74 mg (yield: 82%) of the titled compound as pale yellow powder.
   FAB-MS m/z: 517 [M+H]⁺
   NMR(DMSO-d₆)δ: 3.57(1H, d, J=17.8Hz), 3.61(2H, d, J=6.9Hz), 3.96(1H, d, J=17.8Hz), 5.32(1H, d, J=5.0Hz), 5.89(1H, dd, J=5.0Hz, 8.3Hz), 7.14(1H, s), 7.29-7.38(5H, m), 7.73(1H, d, J=5.3Hz), 8.38(1H, d, J=5.3Hz), 9.37(1H, d, J=8.3Hz)

### Example 2: Preparation of 7-phenylacetamido-3-(6-bromo-7-oxo-7H-thieno[3,2-b]thiopyran-5-yl)thio-3-cephem-4-carboxylic acid (Compound 2)

(1) 7-Phenylacetamido-3-trifluoromethanesulfonyloxy-3-cephem-4-carboxylic acid benzhydryl ester (156 mg, 0.25 mmol) and sodium 5-mercapto-6-bromo-7-oxo-7H-thieno[3,2-b]thiopyran (75 mg, 0.25 mmol) were used to conduct the procedure similar to that shown in (1) of Example 1 to obtain 29 mg (yield: 15%) of 7-phenylacetamido-3-[(6-bromo-7-oxo-7H-thieno[3,2-b]thiopyran-5-yl)thio]-3-cephem-4-carboxylic acid benzhydryl ester as yellow froth.
   NMR(CDCl₃) δ: 3.41(1H, d, J=18.1Hz), 3.69(2H, q, J=9.0Hz), 3.72(1H, d, J=18.1Hz), 5.09(1H, d, J=5.0Hz), 5.99(1H, dd, J=5.0Hz, 9.0Hz), 6.14(1H, d, J=9.0Hz), 6.98(1H, s), 7.11-7.44(16H, m), 7.81(1H, d, J=5.3Hz)
(2) This compound (29 mg, 0.04 mmol) and anisole (118 mg, 0.75 mmol) and trifluoroacetic acid (441 mg, 5.66 mmol) were used to conduct the procedure similar to that shown in (2) of Example 1 to obtain 11 mg (yield: 50%) of the titled compound as pale yellow powder.
   FAB-MS m/z: 596[M+H]⁺
   NMR(DMSO-d₆)δ: 3.56(2H, ABq, J=13.8Hz), 3.66(1H, d, J=17.8Hz), 3.96(1H, d, J=17.8Hz), 5.30(1H, d, J=5.0Hz), 5.86(1H, dd, J=5.0Hz, 8.0Hz), 7.25-7.30(5H, m), 7.58(1H, d, J=5.6Hz), 8.26(1H, d, J=5.6Hz), 9.30(1H, d, J=8.0Hz)

### Example 3: Preparation of 7-phenylacetamido-3-(6-hydoxymethyl-7-oxo-7H-thieno[3,2-b]thiopyran-5-yl)thio-3-cephem-4-carboxylic acid (Compound 3)

(1) 7-Phenylacetamido-3-trifluoromethanesulfonyloxy-3-cephem-4-carboxylic acid benzhydryl ester (195 mg, 0.31 mmol) and sodium 5-mercapto-6-(2-tetrahydropyranyl)oxymethyl-7-oxo-7H-thieno[3,2-b]thiopyran (104 mg, 0.31 mmol) were used to conduct the procedure similar to that shown in (1) of Example 1 to obtain 96 mg (yield: 39%) of 7-phenylacetamido-3-[6-(2-tetrahydropyranyl)oxymethyl-7-oxo-7H-thieno[3,2-b]thiopyran-5-yl]thio-3-cephem-4-carboxylic acid benzhydryl ester (diastereo mixture) as yellow froth.
   NMR(CDCl₃) δ: 1.45-1.80(6H, m), 3.48-3.53(1H, m), 3.58-3.67(4H, m), 3.81-3.91(1H, m), 4.58(0.5H, d, J=10.2Hz), 4.69(0.5H, d, J=10.2Hz), 4.78(1H, s), 4.92(0.5H, d, J=10.2Hz), 4.99(1H, d, J=5.0Hz), 5.02(0.5H, d, J=10.2Hz), 5.90(0.5H, dd, J=5.0Hz, 9.0Hz), 5.91(0.5H, dd, J=5.0Hz, 9.0Hz), 6.03(0.5H, d, J=9.0Hz), 6.05(0.5H, d, J=9.0Hz), 6.97(1H, s), 7.18(1H, d, J=5.3Hz), 7.24-7.42(15H, m), 7.82(1H, d, J=5.3Hz)
(2) This compound (96 mg, 0.12 mmol) and anisole (175 mg, 2.41 mmol) and trifluoroacetic acid (1.38 g, 18.05 mmol) were used to conduct the procedure similar to that shown in (2) of Example 1 to obtain 8 mg (yield: 13%) of the titled compound as pale yellow powder.
   FAB-MS m/z: 546[M+H]⁺
   NMR(DMSO-d₆)δ: 3.52(2H, ABq, J=13.8Hz), 3.81(1H, d, J=17.5Hz), 4.60(1H, d, J=11.5Hz), 4.77(1H, d, J=11.5Hz), 5.20(1H, d, J=5.0Hz), 5.70-5.80(1H, m), 7.17-7.30(5H, m), 7.54(1H, d, J=5.3Hz), 8.23(1H, d, J=5.3Hz), 9.22(1H, d, J=8.2Hz)

### Example 4: Preparation of 7-phenylacetamido-3-(4-oxo-4H-1-benzothiopyran-2-yl)thio-3-cephem-4-carboxylic acid (Compound 4)

(1) 7-Phenylacetamido-3-trifluoromethanesulfonyloxy-3-cephem-4-carboxylic acid benzhydryl ester (160 mg, 0.25 mmol), 2-mercapto-4-oxo-4H-1-benzothiopyran (74 mg, 0.38 mmol) and N-ethyldiisopropylamine (23 mg, 0.17 mmol) were used to conduct the procedure similar to that shown in (1) of Example 1 to obtain 108 mg (yield: 55%) of 7-phenylacetamido-3-(4-oxo-4H-1-benzothiopyran-2-yl)thio-3-cephem-4-carboxylic acid benzhydryl ester as yellow froth.
   NMR(CDCl₃)δ: 3.45(1H, d, J=17.8Hz), 3.65(1H, d, J=17.8Hz), 3.66(2H, d, J=3.0Hz), 5.05(1H, d, J=5.0Hz), 5.92(1H, dd, J=5.0Hz, 9.2Hz), 6.43(1H, d, J=8.9Hz), 6.94(1H, s), 6.99(1H, s), 7.15-7.62(18H, m), 8.47(1H, d, J=9.2Hz)
(2) This compound (105 mg, 0.15 mmol) and anisole (355 mg, 3.10 mmol) and trifluoroacetic acid (353 mg, 3.10 mmol) were used to conduct the procedure similar to that shown in (2) of Example 1 to obtain 27 mg (yield: 34%) of the titled compound as pale yellow powder.
   FAB-MS m/z: 511[M+H]⁺
   NMR(DMSO-d₆)δ: 3.55(1H, d, J=17.8Hz), 3.62(2H, d, J=6.9Hz), 3.99(1H, d, J=17.8Hz), 5.34(1H, d, J=5.0Hz), 5.91(1H, dd, J=5.0Hz, 8.3Hz), 7.09(1H, s), 7.20-7.55(5H, m), 7.69-7.94(3H, m), 8.40(1H, d, J=7.9Hz), 9.39(1H, d, J=8.3Hz)

### Example 5: Preparation of 7-phenylacetamido-3-(5-aminomethyl-2-chloro-4-oxo-4H-thieno[2,3-b]thiopyran-6-yl)thio-3-cephem-4-carboxylic acid (Compound 5)

(1) 7-Phenylacetamido-3-trifluoromethanesulfonyloxy-3-cephem-4-carboxylic acid benzhydryl ester (100 mg, 0.158 mmol), 5-tert-butoxycarbonylaminomethyl-2-chloro-4-oxo-6-mercapto-4H-thieno[2,3-b]thiopyran (63 mg, 0.19 mmol) and N-ethyldiisopropylamine (16.3 mg, 0.126 mmol) were used to conduct the procedure similar to that shown in (1) of Example 1 to obtain 40 mg (yield: 30%) of 7-phenylacetamido-3-(5-tert-butoxycarbonylaminomethyl-2-chloro-4-oxo-4H-thieno[2,3-b]thiopyran-6-yl)thio-3-cephem-4-carboxylic acid benzhydryl ester as yellow froth.
   NMR(CDCl₃)δ: 3.48(1H, d, J=18.0Hz), 3.64(3H, ABq+d, J=16.0Hz, 18.0Hz), 4.46(2H, brs), 5.03(1H, d, J=5.0Hz), 5.56(1H, br), 5.90(1H, dd, J=5.0Hz, 9.0Hz), 6.04(1H, d, J=9.0Hz), 6.96(1H, s), 7.24-7.38(15H, m), 7.50(1H, s)
(2) This compound (30 mg, 0.035 mmol) and anisole (75.7 mg, 0.7 mmol) and trifluoroacetic acid (600 mg, 5.25 mmol) were used to conduct the procedure similar to that shown in (2) of Example 1 to obtain 17 mg (yield: 70%) of the titled compound as pale yellow powder.
   FAB-MS m/z: 580[M+H]⁺
   NMR(DMSO-d₆)δ: 3.55(3H, m), 3.80(1H, d, J=17.5Hz), 4.18(2H, brs), 5.18(1H, d, J=4.6Hz), 5.75(1H, d, J=4.6Hz, 8.3Hz), 7.26-7.33(5H, m), 9.16(1H, d, J=8.3Hz)

### Example 6: Preparation of 7-phenylacetamido-3-(7-oxo-7H-thiopyrano[3,2-b]furan-5-yl)thio-3-cephem-4-carboxylic acid (Compound 6)

(1) 7-Phenylacetamido-3-trifluoromethanesulfonyloxy-3-cephem-4-carboxylic acid benzhydryl ester (200 mg, 0.316 mmol), 5-mercapto-7-oxo-7H-1-thiopyrano[3,2-b]furan (69.9 mg, 0.38 mmol) and N-ethyldiisopropylamlne (32.7 mg, 0.25 mmol) were used to conduct the procedure similar to that shown in (1) of Example 1 to obtain 92 mg (yield: 43.5%) of 7-phenylacetamido-3-(7-oxo-7H-1-thiopyrano[3,2-b]furan-5-yl)thio-3-cephem-4-carboxylic acid benzhydryl ester.
   NMR(CDCl₃)δ: 3.41(1H, d, J=18.0Hz), 3.60(1H, d, J=18.0Hz), 3.65(2H, ABq, J=16.0Hz), 5.02(1H, d, J=5.0Hz), 5.90(1H, dd, J=5.0Hz, 9.0Hz), 6.58(1H, d, J=9.0Hz), 6.74(1H, d, J=2.0Hz), 6.97(1H, s), 7.12(1H, s), 7.21-7.37(15H, m), 7.85(1H, d, J=2.0Hz)
(2) This compound (77 mg, 0.115 mmol), anisole (872 mg, 8.06 mmol) and trifluoroacetic acid (1.57 g, 13.8 mmol) were used to conduct the procedure similar to that shown in (2) of Example 1 to obtain 25 mg (yield: 43.5%) of the titled compound as pale yellow powder.
   FAB-MS m/z: 501[M+H]⁺
   NMR(DMSO-d₆)δ: 3.50-3.66(3H, m), 3.84(1H, d, J=17.8Hz), 5.22(1H, d, J=5.0Hz), 5.79(1H, dd, J=5.0Hz, 8.2Hz), 7.17(1H, s), 7.20(1H, d, J=2.0Hz), 7.23-7.36(5H, m), 8.28(1H, d, J=2.0Hz), 9.27(1H, d, J=8.2Hz)

### Example 7: Preparation of 7-phenylacetamido-3-(2-chloro-4-oxo-4H-thieno[2,3-b]thiopyran-6-yl)thio-3-cephem-4-carboxylic acid (Compound 7)

(1) 7-Phenylacetamido-3-trifluoromethanesulfonyloxy-3-cephem-4-carboxylic acid benzhydryl ester (300 mg, 0.47 mmol), 2-chloro-4-oxo-6-mercapto-4H-thieno[2,3-b]thiopyran (121 mg, 0.52 mmol) and N-ethyldiisopropylamine (48.6 mg, 0.38 mmol) were used to conduct the procedure similar to that shown in (1) of Example 1 to obtain 170 mg (yield: 50.4%) of 7-phenylacetamido-3-(2-chloro-4-oxo-4H-thieno[2,3-b]thiopyran-6-yl)thio-3-cephem-4-carboxylic acid benzhydryl ester.
   NMR(CDCl₃)δ: 3.37(1H, d, J=17.8Hz), 3.60(1H, d, J=17.8Hz), 3.6 4(2H, ABq, J=16.0Hz), 5.02(1H, d, J=5.2Hz), 5.90(1H, dd, J=5.0Hz, 8.9Hz), 6.29(1H, d, J=8.9Hz), 6.98(1H, s), 7.22-7.40(15H, m), 7.52(1H, s)
(2) This compound (140 mg, 0.195 mmol), anisole (1.48 g, 13.66 mmol) and trifluoroacetic acid (2.67 g, 23.4 mmol) were used to conduct the procedure similar to that shown in (2) of Example 1 to obtain 50 mg (yield: 46%) of the titled compound as pale yellow powder.
   FAB-MS m/z: 551[M+H]⁺
   NMR(DMSO-d₆)δ: 3.61(2H, ABq, J=14.0Hz), 3.70(1H, d, J=18.0Hz), 3.93(1H, d, J=18.0Hz), 5.30(1H, d, J=4.6Hz), 5.88(1H, dd, J=4. 6Hz, 8.0Hz), 7.16(1H, s), 7.35(5H, s), 7.69(1H, s), 9.32(1H, d, J=8.0Hz)

### Example 8: Preparation of 7-phenylacetamido-3-(4-oxo-4H-thiopyrano[3,2-c]pyridin-2-yl)thio-3-cephem-4-carboxylic acid (Compound 8)

(1) 7-Phenylacetamido-3-trifluoromethanesulfonyloxy-3-cephem-4-carboxylic acid benzhydryl ester (230 mg, 0.36 mmol), 2-mercapto-4-oxo-4H-thieno[3,2-c]pyridine (78 mg, 0.40 mmol) and N-ethyldiisopropylamine (26 mg, 0.28 mmol) were used to conduct the procedure similar to that shown in (1) of Example 1 to obtain 148 mg (yield: 60%) of 7-phenylacetamido-3-(4-oxo-4H-thiopyrano[3,2-c]pyridin-2-yl)thio-3-cephem-4-carboxylic acid benzhydryl ester as yellow froth.
   NMR(CDCl₃)δ: 3.40(1H, d, J=18.1Hz), 3.66(2H, s), 3.70(1H, d, J=18.1Hz), 5.07 (1H, d, J=5.0Hz), 5.96(1H, dd, J=5.0Hz, 9.0Hz), 6.36(1H, d, J=9.0Hz), 6.93(1H, s), 6.99(1H, s), 7.17-7.41(16H, m), 8.68(1H, d, J=5.3Hz), 9.55(1H, s)
(2) This compound (64 mg, 0.095 mmol), anisole (20 mg, 1.90 mmol) and trifluoroacetic acid (1.581 g, 14.22 mmol) were used to conduct the procedure similar to that shown in (2) of Example 1 to obtain 47 mg (yield: 96%) of the titled compound as pale yellow powder.
   FAB-MS m/z: 512[M+H]⁺
   NMR(DMSO-d₆)δ: 3.54(2H, ABq, J=13.8Hz), 3.69(1H, d, J=17.8Hz), 3.92(1H, d, J=17.8Hz), 5.26(1H, d, J=5.0Hz), 5.84(1H, dd, J=5.0Hz, 9.0Hz), 7.06(1H, s), 7.19-7.32(5H, m), 7.85(1H, d, J=5.6Hz), 8.73(1H, d, J=5.6Hz), 9.31(1H, d, J=9.0Hz), 9.35(1H, s)

### Example 9: Preparation of 7-phenylacetamido-3-(4-oxo-4H-thiopyrano[3,2-b]pyridin-2-yl)thio-3-cephem-4-carboxylic acid (Compound 9)

(1) 7-Phenylacetamido-3-trifluoromethanesulfonyloxy-3-cephem-4-carboxylic acid benzhydryl ester (1.47 g, 2.33 mmol), 2-mercapto-4-oxo-4H-thiopyrano[3,2-b]pyridine (500 mg, 2.56 mmol) and N-ethyldiisopropylamine (240.9 mg, 1.86 mmol) were used to conduct the procedure similar to that shown in (1) of Example 1 to obtain 420 mg (yield: 26.6%) of 7-phenylacetamido-3-(4-oxo-4H-thiopyrano[3,2-b]pyridin-2-yl)thio-3-cephem-4-carboxylic acid benzhydryl ester as yellow powder.
   NMR(CDCl₃)δ: 3.43(1H, d, J=18.0Hz), 3.66(3H, s+d, J=18.0Hz), 5.06(1H, d, J=5.2Hz), 5.93(1H, dd, J=5.2Hz, 9.0Hz), 6.54(1H, d, J=9.0Hz), 6.98(1H, s), 7.06(1H, s), 7.14-7.39(15H, m), 7.53(1H, dd, J=4.6Hz, 8.2Hz), 7.85(1H, dd, J=1.6Hz, 8.2Hz), 8.94(1H, dd, J=1.6Hz, 4.6Hz)
(2) This compound (60 mg, 0.088 mmol), anisole (191.5 mg, 1.77 mmol) and trifluoroacetic acid (1.2 g, 10.56 mmol) were used to conduct the procedure similar to that shown in (2) of Example 1 to obtain 40 mg (yield: 89%) of the titled compound as pale yellow powder.
   FAB-MS m/z: 512[M+H]⁺
   NMR(DMSO-d₆)δ: 3.53(2H, ABq, J=13.8Hz), 3.64(1H, d, J=17.8Hz), 3.91(1H, d, J=17.8Hz), 5.25(1H, d, J=4.9Hz), 5.81(1H, dd, J=4.9Hz, 8.5Hz), 7.11(1H, s), 7.19-7.32(5H, m), 7.72(1H, dd, J=4.2Hz, 8.5Hz), 8.32(1H, dd, J=1.3Hz, 8.5Hz), 8.87(1H, d, J=4.2Hz), 9.30(1H, d, J=8.5Hz)

### Example 10: Preparation of 7-phenylacetamido-3-(7-isothiuroniummethyl-4-oxo-4H-1-benzothiopyran-2-yl)thio-3-cephem-4-carboxylic acid · trifluoro acetate (Compound 10)

(1) 7-Phenylacetamido-3-trifluoromethanesulfonyloxy-3-cephem-4-carboxylic acid benzhydryl ester (400 mg, 0.63 mmol), 7-hydroxymethyl-2-mercapto-7-oxo-4H-1-benzothiopyrano (148 mg, 0.66 mmol) and N-ethyldiisopropylamine (48.9 mg, 0.38 mmol) were used to conduct the procedure similar to that shown in (1) of Example 1 to obtain 60 mg (yield: 13.5%) of 7-phenylacetamido-3-(7-hydroxymethyl-4-oxo-4H-1-benzothiopyran-2-yl)thio-3-cephem-4-carboxylic acid benzhydryl ester.
   NMR(CDCl₃)δ: 3.54(2H, ABq, J=13.5Hz), 3.65(1H, d, J=17.8Hz), 3.93(1H, d, J=17.8Hz), 4.66(2H, d, J=5.2Hz), 5.31(1H, d, J=5.2H z), 5.53(1H, t, J=5.2Hz), 5.90(1H, dd, J=4.9Hz, 8.5Hz), 6.82(1H, s), 6.99(1H, s), 7.09-7.35(15H, m), 7.54(1H, d, J=8.2Hz), 7.66(1H, s), 8.26(1H, d, J=8.2Hz), 9.36(1H, d, J=8.5Hz)
(2) N,N-Dimethylformamide (2.5 ml) was added dropwise with thionyl chloride (101 mg, 0.85 mmol) under ice-water cooling and stirred at the same temperature for 30 minutes. Then, the above-mentioned obtained compound (60 mg, 0.085 mmol) was added to the reaction mixture and stirred further at the same tempeature for 1 hour. The resultant mixture was added with ice water (20 ml) and the ocher precipitates were collected, fully washed with ice water and dried under reduced pressure to obtain 36 mg (yield: 58.4%) of 7-phenylacetamido-3-(7-chloromethyl-4-oxo-4H-1-benzothiopyran-2-yl)thio-3-cephem-4-carboxylic acid benzhydryl ester.
   NMR(CDCl₃)δ: 3.42(1H, d, J=17.8Hz), 3.66(2H, s), 3.68(1H, d, J=17.8Hz), 4.65(2H, s), 5.05(1H, d, J=4.9Hz), 5.92(1H, dd, J=4.9Hz, 9.2Hz), 6.39(1H, d, J=9.2Hz), 6.94(1H, s), 6.98(1H, s), 7.09-7.40(15H, m), 7.54(2H, s+d, J=8.9Hz), 8.44(1H, d, J=8.9Hz)
(3) The obtained compound (120 mg, 0.165 mmol) and thiourea (188.4 mg, 2.48 mmol) were stirred in ethanol (10 ml) at room temperature for 4 days and the solvent was removed under reduced pressure. The residue was dissolved in methanol (6 ml) and the insoluble matter was filtered. The filtrate was dealt with Cephadex LH-20 (methanol) to separate the main component. The main component was crystallized from ethyl acetate to obtain 63 mg (yield: 48%) of 7-phenylacetamido-3-(7-isothiuroniummethyl-4-oxo-4H-1-benzothiopyran-2-yl)thio-3-cephem-4-carboxylic acid benzhydryl ester · hydrochloride.
   FAB-MS m/z: 765[M-HCl+H]⁺
   NMR(CDCl₃)δ: 3.54(2H, ABq, J=14.0Hz), 3.65(1H, d, J=17.8Hz), 3.94(1H, d, J=17.8Hz), 4.64(2H, s), 5.31(1H, d, J=5.0Hz), 5.90 (1H, dd, J=5.0Hz, 8.5Hz), 6.83(1H, s), 6.98(1H, s), 7.12-7.36(15H, m), 7.66(1H, d, J=8.2Hz), 7.80(1H, s), 8.30(1H, d, J=8.2Hz), 9.11(1H, br), 9.31(2H, br), 9.38(1H, d, J=8.5Hz)
(4) This compound (50 mg, 0.062 mmol) and anisole (472.3 mg, 4.37 mmol) and trifluoroacetic acid (848 mg, 7.44 mmol) were used to conduct the procedure similar to that shown in (2) of Example 1 to obtain 38 mg (yield: 86%) of the titled compound as pale yellow powder.
   FAB-MS m/z: 599[M+H]⁺
   NMR(DMSO-d₆)δ: 3.40(1H, d, J=18.0Hz), 3.53(2H, ABq, J=13.5Hz), 3.88(1H, d, J=18.0Hz), 4.62(2H, s), 5.23(1H, d, J=5.0Hz), 5.78 (1H, dd, J=5.0Hz, 8.2Hz), 6.97(1H, s), 7.20-7.28(5H, m), 7.64(1H, d, J=8.0Hz), 7.83(1H, s), 8.29(1H, d, J=8.0Hz), 9.30(4H, d+br, J=8.2Hz)

### Example 11: Preparation of 7-phenylacetamido-3-(6-chloro-7-oxo-7H-thieno[3,2-b]thiopyran-5-yl)thio-3-cephem-4-carboxylic acid benzhydryl ester

(1) 7-Phenylacetamido-3-trifluoromethanesulfonyloxy-3-cephem-4-carboxylic acid benzhydryl ester 1-oxide (1.31 g, 2.04 mmol) dissolved in N,N-dimethylformamide (15 ml) was cooled to -15 to -20°C, added dropwise with sodium 6-chloro-5-mercapto-7-oxo-7H-thieno[3,2-b]thiopyran (2.27 mmol) dissolved in tetrahydrofuran and stirred at the same temperature for 15 minutes. The resultant mixture was poured into ice water and the precipitating precipitates were filtered out to obtain 1.1 g (yield: 74%) of 7-phenylacetamido-3-(6-chloro-7-oxo-7H-thieno[3,2-b]thiopyran-5-yl)thio-3-cephem-4-carboxylic acid benzhydryl ester 1-oxide.
   NMR(CDCl₃)δ: 3.28(1H, d, J=18.0Hz), 3.64(2H, ABq, J=15.5Hz), 3.85(1H, d, J=18.0Hz), 4.55(1H, d, J=4.9Hz), 6.16(1H, dd, J=4.9Hz, 9.9Hz), 6.71(1H, d, J=9.9Hz), 6.97(1H, s), 7.15-7.39(16H, m), 7.82(1H, d, J=5.2Hz)
(2) The obtained compound (1 g, 1.36 mmol) was dissolved in N,N-dimethylformamide (10 ml), cooled to -30°C, added with phosphorus trichloride (466.9 mg, 3.4 mmol) and stirred at the same temperature for 1.5 minutes. The resultant mixture was added with ethyl acetate-water (1 : 1) (100 ml), stirred for a while and further added with ethyl acetate (100 ml) to separate an ethyl acetate layer. The ethyl acetate layer was washed with 5% aqueous solution of sodium hydrogencarbonate and saturated saline solution, successively, and dried over anhydrous magnesium sulfate. The solvent was removed under reduced pressure and the residue was purified by column chromatography (Wako Gel™ C-200 : dichloromethane-ethyl acetate = 8 : 1) and crystallized by ether to obtain 570 mg (yield: 58.4%) of the titled compound as yellow powder.
   NMR(CDCl₃)δ: 3.39(1H, d, J=18.0Hz), 3.68(2H, ABq, J=16.0Hz), 3.70(1H, d, J=18.0Hz), 5.09(1H, d, J=5.0Hz), 5.96(1H, dd, J=5.0Hz, 9.0Hz), 6.10(1H, d, J=9.0Hz), 6.98(1H, s), 7.13-7.41(16H, m), 7.82(1H, d, J=5.2Hz)

The following compounds were obtained by the procedure similar to that shown in the Example 11 from the corresponding starting materials.
- 7-Phenylacetamido-3-[6-[2-(tert-butoxycarbonyl)aminoethylthiomethyl]-7-oxo-7H-thieno[3,2-b]thiopyran-5-yl]thio-3-cephem-4-carboxylic acid benzhydryl ester
   NMR(CDCl₃)δ: 1.43(9H, s), 3.30(2H, t, J=5.3Hz), 3.44(1H, d, J=18.1Hz), 3.64(2H, d, J=3.9Hz), 3.64-3.75(3H, m), 4.63(2H, dd, J=14.2Hz, 15.8Hz), 5.05(1H, d, J=5.0Hz), 5.92(1H, dd, J=5.0Hz, 8.9Hz), 6.14(1H, d, J=8.9Hz), 6.97(1H, s), 7.20-7.39(16H, m), 7.88(1H, d, J=5.3Hz)
- 7-Phenylacetamido-3-(6-methoxymethyloxymethyl-7-oxo-7H-thieno[3,2-b]-thiopyran-5-yl)thio-3-cephem-4-carboxylic acid benzhydryl ester
   NMR(CDCl₃)δ: 3.35(3H, s), 3.46(1H, d, J=17.8Hz), 3.61(1H, d, J=16.2 Hz), 3.65(1H, d, J=17.8Hz), 3.69(1H, d, J=16.2Hz), 4.66(2H, s), 4.74 (2H, d, J=3.9Hz), 5.02(1H, d, J=5.0Hz), 5.89(1H, dd, J=5.0Hz, 8.9Hz), 6.08(1H, d, J=8.9Hz), 6.98(1H, s), 7.18(1H, d, J=5.3Hz), 7.21-7.39 (15H, m), 7.82(1H, d, J=5.3Hz)
- 7-Phenylacetamido-3-(6-chloro-7-oxo-7H-1-thiopyrano[3,2-b]furan-5-yl)thio-3-cephem-4-carboxylic acid benzhydryl ester
   NMR(CDCl₃)δ: 3.37(1H, d, J=17.8Hz), 3.67(1H, d, J=17.8Hz), 3.68(2H, ABq, J=16.2Hz), 5.07(1H, d, J=5.3Hz), 5.96(1H, dd, J=5.3Hz, 8.9Hz), 6.18 (1H, d, J=8.9Hz), 6.73(1H, d, J=2.0Hz), 6.97(1H, s), 7.13-7.42(15H, m), 7.83(1H, d, J=2.0Hz)
- 7-Phenylacetamido-3-(3-bromo-7-oxo-7H-thieno[3,2-b]thiopyran-5-yl)thio-3-cephem-4-carboxylic acid benzhydryl ester
   NMR(CDCl₃)δ: 3.40(1H, d, J=17.8Hz), 3.65(1H, d, J=17.8Hz), 3.66(2H, ABq, J=16.0Hz), 5.04(1H, d, J=5.0Hz), 5.93(1H, dd, J=5.0 Hz, 9.0Hz), 6.42(1H, d, J=9.0Hz), 6.98(1H, s), 6.99(1H, s), 7.20-7.40(15H, m), 7.81(1H, s)
- 7-Phenylacetamido-3-(2-bromo-4-oxo-4H-thieno[2,3-b]thiopyran-6-yl)thio-3-cephem-4-carboxylic acid benzhydryl ester
   NMR(CDCl₃)δ: 3.38(1H, d, J=18.0Hz), 3.62(1H, d, J=18.0Hz), 3.65(2H, ABq, J=16.0Hz), 5.03(1H, d, J=5.0Hz), 5.48(1H, dd, J=5.0Hz, 9.0Hz), 6.34(1H, d, J=9.0Hz), 6.94(1H, s), 6.98(1H, s), 7.20-7.40(16H, m)

### Example 12: Preparation of 7-[2-(2-thienyl)acetamido]-3-(7-oxo-7H-thieno[3,2-b]thiopyran-5-yl)thio-3-cephem-4-carboxylic acid (Compound 11)

(1) Phosphorous pentachloride (183 mg, 0.87 mmol) suspended in anhydrous dichloromethane (5.0 ml) was added with pyridine (69 mg, 0.87 mmol) at the temperature of -10°C under nitrogen atmosphere and stirred at the same temperature for 30 minutes. The reaction mixture was added dropwise with 7-phenylacetamido-3-(7-oxo-7H-thieno[3,2-b]thiopyran-5-yl)thio-3-cephem-4-carboxylic acid benzhydryl ester (400 mg, 0.58 mmol) obtained in (1) of the Example 1 and dissolved in anhydrous dichloromethane (8.0 ml). The reaction mixture was stirred at the temperature range of -20 to -10°C for 4 hours. The resultant mixture was added with methyl cellosolve (445 mg, 5.85 mmol) and further stirred at the same temperature for 1 hour. Then, the mixture was added with ice water (10 g) and stirred violently for 5 minutes. The reaction mixture was added with an appropriate amount of aqueous solution of saturated sodium hydrogencarbonate to be adjusted to pH 7.5-8, and extracted with ethyl acetate (80 ml × 2 times). The ethyl acetate layer was washed with saturated saline solution and dried over anhydrous magnesium sulfate. The solvent was removed under reduced pressure and the residue was crystallized from isopropylether to obtain 303 mg (yield: 92%) of 7*β*-amino-3-(7-oxo-7H-thieno[3,2-b]thiopyran-5-yl)thio-3-cephem-4-carboxylic acid benzhydryl ester as crude crystals.
(2) The obtained compound (100 mg, 0.177 mmol) was dissolved in dichloromethane (10 ml), added with thiophen-2-acetic acid (50 mg, 0.354 mmol) and dicyclohexylcarbodiimide (73 mg, 0.354 mmol) and stirred at room temperature for 4 hours. The product was diluted with ethyl acetate (20 ml) and the insoluble matter was filtered. The filtrate was removed under reduced pressure and the residue was purified by column chromatography [Wako Gel™ C-200 : hexane-ethyl acetate(1 : 2)] to obtain 78 mg (yield: 64%) of 7-[2-(2-thienyl)acetamido]-3-(7-oxo-7H-thieno[3,2-b]thiopyran-5-yl)thio-3-cephem-4-carboxylic acid benzhydryl ester as yellow froth.
   NMR(CDCl₃)δ: 3.42(1H, ABq, J=17.8Hz), 3.64(1H, ABq, J=17.8Hz), 3.87(2H, s), 5.04(1H, d, J=5.0Hz), 5.89(1H, dd, J=5.0Hz, 9.2Hz), 6.79(1H, d, J=9.2Hz), 6.94-7.02(5H, m), 7.17-7.35(11H, m), 7.86(1H, d, J=5.3Hz)
(3) This compound (78 mg, 0.11 mmol), anisole (244 mg, 2.26 mmol) and trifluoroacetic acid (257 mg, 2.26 mmol) were used to conduct the procedure similar to that shown in (2) of Example 1 to obtain 15 mg (yield: 25%) of the titled compound as pale yellow powder.
   FAB-MS m/z: 523[M+H]⁺
   NMR(DMSO-d₆)δ: 3.62(1H, d, J=17.8Hz), 3.75(2H, s), 3.87(1H, d, J=17.8Hz), 5.24(1H, d, J=5.0Hz), 5.81(1H, dd, J=5.0Hz, 8.3Hz), 6.85-6.94(2H, m), 7.07 (1H, s), 7.35(1H, dd, J=1.3Hz, 4.9Hz), 7.62(1H, d, J=5.3Hz), 8.28(1H, d, J=5.3Hz), 9.31(1H, d, J=8.3Hz), 14.20(1H, brs)

### Example 13: Preparation of 7-[2-hydroxyimino-2-(2-aminothiazol-4-yl)acetamido]-3-(7-oxo-7H-thieno[3,2-b]thiopyran-5-yl)thio-3-cephem-4-carboxylic acid (Compound 12)

(1) 7*β*-amino-3-(7-oxo-7H-thieno[3,2-b]thiopyran-5-yl)thio-3-cephem-4-carboxylic acid benzhydryl ester (100 mg, 0.177 mmol) obtained in (1) of the Example 12, (Z)-trityloxyimino-2-(2-tritylaminothiazol-4-yl)acetic acid (237 mg, 0.354 mmol) and dicyclohexylcarbodiimide (73 mg, 0.354 mmol) were used to conduct the procedure similar to that shown in (2) of Example 12 to obtain 62 mg (yield: 28%) of 7-[2-trityloxyimino-2-(2-tritylaminothiazol-4-yl)acetamido]-3-(7-oxo-7H-thieno[3,2-b]thiopyran-5-yl)thio-3-cephem-4-carboxylic acid benzhydryl ester as brown froth.
   NMR(CDCl₃)δ: 3.25(1H, d, J=17.8Hz), 3.61(1H, d, J=17.8Hz), 5.15(1H, d, J=5.0Hz), 6,12(1H, dd, J=5.0Hz, 8.9Hz), 6.42(1H, s), 6.73(1H, brs), 7.03(1H, s), 7.11(1H, s), 7.14(1H, d, J=5.3Hz), 7.19-7.14(41H, m), 7.81(1H, d, J=5.3Hz)
(2) This compound (62 mg, 0.05 mmol), anisole (110 mg, 1.01 mmol) and trifluoroacetic acid (872 mg, 7.62 mmol) were used to conduct the procedure similar to that shown in (2) of Example 1 to obtain 17 mg (yield: 48%) of the titled compound as pale yellow powder.
   FAB-MS m/z: 568[M+H]⁺
   NMR(DMSO-d₆)δ: 3.66(1H, d, J=17.5Hz), 3.93(1H, d, J=17.5Hz), 5.37(1H, d, J=5.0Hz), 5.96(1H, dd, J=5.0Hz, 8.3Hz), 6.76(1H, s), 7.11(1H, s), 7.69(1H, d, J=5.3Hz), 8.34(1H, d, J=5.3Hz), 9.72(1H, d, J=5.3Hz), 11.62(1H, brs)

### Example 14: Preparation of 7-[2-hydroxyimino-2-(2-amino-5-chlorothiazol-4-yl)acetamido]-3-(7-oxo-7H-thieno[3,2-b]thiopyran-5-yl)thio-3-cephem-4-carboxylic acid (Compound 13)

(1) 7*β*-amino-3-(7-oxo-7H-thieno[3,2-b]thiopyran-5-yl)thio-3-cephem-4-carboxylic acid benzhydryl ester (250 mg, 0.44 mmol) obtained in (1) of Example 12, (Z)-trityloxyimino-2-(2-tritylamino-5-chlorothiazol-4-yl)acetic acid (625 mg, 0.88 mmol) and dicyclohexylcarbodiimide (182 mg, 0.88 mmol) were used to conduct the procedure similar to that shown in (2) of Example 12 to obtain 205 mg (yield: 37%) of 7-[2-trityloxylmino-2-(2-tritylamino-5-chlorothiazol-4-yl)acetamido]-3-(7-oxo-7H-thieno[3,2-b]thiopyran-5-yl)thio-3-cephem-4-carboxylic acid benzhydryl ester as brown froth.
   NMR(CDCl₃)δ: 3.21(1H, d, J=17.8Hz), 3.59(1H, d, J=17.8Hz), 5.15(1H, d, J=5.3Hz), 6.14(1H, dd, J=5.3Hz, 9.2Hz), 6.59(1H, s), 7.01(1H, s), 7.03(1H, s), 7.19-7.41(41H, m), 7.81(1H, d, J=5.3Hz)
(2) This compound (105 mg, 0.16 mmol), anisole (344 mg, 3.18 mmol) and trifluoroacetic acid (2.72 g, 23.85 mmol) were used to conduct the procedure similar to that shown in (2) of Example 1 to obtain 28 mg (yield: 25%) of the titled compound as pale yellow powder.
   FAB-MS m/z: 602[M+H]⁺
   NMR(DMSO-d₆)δ: 3.30(1H, d, J=17.5Hz), 3.71(1H, d, J=17.5Hz), 5.15(1H, d, J=5.3Hz), 5.70(1H, dd, J=5.3Hz, 8.3Hz), 6.96(1H, s), 7.58(1H, d, J=5.8Hz), 8.22(1H, d, J=5.3Hz), 9.51(1H, d, J=8.3Hz)

### Example 15: Preparation of 7-[2-hydroxyimino-2-(2-amino-5-chlorothiazol-4-yl)acetamido]-3-(6-bromo-7-oxo-7H-thieno[3,2-b]thiopyran-5-yl)thio-3-cephem-4-carboxylic acid (Compound 14)

(1) 7-Pheylacetamido-3-(6-bromo-7-oxo-7H-thieno[3,2-b]thiopyran-5-yl)thio-3-cephem-4-carboxylic acid benzhydryl ester (962 mg, 1.26 mmol), phosphorous pentachloride (526 mg, 2.52 mmol), pyridine (199 mg, 2.52 mmol) and methyl cellosolve (0.91 g, 12.60 mmol) were used to conduct the procedure similar to that shown in (1) of Example 12 to obtain 500 mg (yield: 61%) of 7*β*-amino-3-(6-bromo-7-oxo-7H-thieno[3,2-b]thiopyran-5-yl)thio-3-cephem-4-carboxylic acid benzhydryl ester as crude crystals.
(2) The obtained compound (500 mg, 0.77 mmol), (Z)-2-trityloxyimino-2-(2-tritylamino-5-chlorothiazol-4-yl)acetic acid (1097 mg, 1.55 mmol) and dicyclohexylcarbodiimide (320 mg, 1.55 mmol) were used to conduct the procedure similar to that shown in (2) of Example 12 to obtain 513 mg (yield: 50%) of 7-[2-trityloxyimino-2-(2-tritylamino-5-chlorothiazol-4-yl)acetamido]-3-(6-bromo-7-oxo-7H-thieno[3,2-b]thiopyran-5-yl)thio-3-cephem-4-carboxylic acid benzhydryl ester as brown froth.
   NMR(CDCl₃)δ: 3.26(1H, d, J=18.1Hz), 3.69(1H, d, J=18.1Hz), 5.22(1H, d, J=5.3Hz), 6.19(1H, dd, J=5.3Hz, 9.2Hz), 6.63(1H, s), 6.96-7.39(43H, m), 7.44(1H, d, J=5.3Hz)
(3) This compound (200 mg, 0.15 mmol), anisole (324 mg, 3.00 mmol) and trifluoroacetic acid (2.56 g, 22.5 mmol) were used to conduct the procedure similar to that shown in (2) of Example 1 to obtain 39 mg (yield: 32%) of the titled compound as pale yellow powder.
   FAB-MS m/z: 682[M+H]⁺
   NMR(DMSO-d₆)δ: 3.36(1H, d, J=17.5Hz), 3.77(1H, d, J=17.5Hz), 5.17(1H, d, J=5.3Hz), 5.73(1H, dd, J=5.3Hz, 8.6Hz), 7.49(1H, d, J=5.8Hz), 8.05(1H, d, J=5.3Hz), 9.43(1H, d, J=8.6Hz)

### Example 16: Preparation of 7-[2-hydroxyimino-2-(2-amino-5-chlorothiazol-4-yl)acetamido]-3-(6-chloro-7-oxo-7H-thieno[3,2-b]thiopyran-5-yl)thio-3-cephem-4-carboxylic acid (Compound 15)

(1) 7-Phenylacetamido-3-(6-chloro-7-oxo-7H-thieno[3,2-b]thiopyran-5-yl)thio-3-cephem-4-carboxylic acid benzhydryl ester (570 mg, 0.795 mmol), phosphorous pentachloride (331 mg, 1.59 mmol), pyridine (125.8 mg, 1.59 mmol) and methanol (3.2 g, 98.9 mmol) were used to conduct the procedure similar to that shown in (1) of Example 12 to obtain 7*β*-amino-3-(6-chloro-7-oxo-7H-thieno[3,2-b]thiopyran-5-yl)thio-3-cephem-4-carboxylic acid benzhydryl ester as crude crystals.
(2) The obtained crude crystals, (Z)-trityloxyimino-2-(2-tritylamino-5-chlorothiazol-4-yl)acetic acid (645.7 mg, 0.914 mmol) and dicyclohexylcarbodiimide (189 mg, 0.914 mmol) were used to conduct the procedure similar to that shown in (2) of Example 16 to obtain 100 mg (yield: 10%) of 7-[2-trityloxyimino-2-(2-tritylamino-5-chlorothiazol-4-yl)acetamido]-3-(6-chloro-7-oxo-7H-thieno[3,2-b]thiopyran-5-yl)thio-3-cephem-4-carboxylic acid benzhydryl ester as yellow powder.
   NMR(CDCl₃)δ: 3.24(1H, d, J=18.0Hz), 3.67(1H, d, J=18.0Hz), 5.21(1H, d, J=5.2Hz), 6.18(1H, dd, J=5.2Hz, 9.5Hz), 6.62(1H, s), 6.98(1H, d, J=9.5Hz), 7.01(1H, s), 7.04(1H, d, J=5.2Hz), 7.10-7.42(40H, m), 7.76(1H, d, J=5.2Hz)
(3) This compound (90 mg, 0.07 mmol), anisole (151.4 mg, 1.40 mmol) and trifluoroacetic acid (1.44 g, 12.6 mmol) were used to conduct the procedure similar to that shown in (2) of Example 1 to obtain 16 mg (yield: 30%) of the titled compound as pale yellow powder.
   FAB-MS m/z: 636[M+H]⁺
   NMR(DMSO-d₆)δ: 3.61(1H, d, J=17.8Hz), 3.91(1H, d, J=17.8Hz), 5.33(1H, d, J=5.2Hz), 5.95(1H, dd, J=5.2Hz, 8.2Hz), 7.26(2H, br), 7.58(1H, d, J=5.2Hz), 8.25(1H, d, J=5.2Hz), 9.55(1H, d, J=8.2Hz), 11.73(1H, s)

### Example 17: Preparation of 7-[2-hydroxyimino-2-(2-amino-5-chlorothiazol-4-yl)acetamido]-3-[6-(2-aminoethylthiomethyl)-7-oxo-7H-thieno[3,2-b]thiopyran-5-yl]thio-3-cephem-4-carboxylic acid (Compound 16)

(1) 7-Phenylacetoamido-3-[6-[2-(tert-butoxycarbonyl)aminoethylthiomethyl]-7-oxo-7H-thieno[3,2-b]thiopyran-5-yl]thio-3-cephem-4-carboxylic acid benzhydryl ester (512 mg, 0.58 mmol) obtained the procedure similar to that shown in Example 11, phosphorous pentachloride (244 mg, 1.17 mmol), pyridine (93 mg, 1.17 mmol) and methyl cellosolve (447 mg, 5.87 mmol) were used to conduct the procedure similar to that shown in (1) of Example 12 to obtain 305 mg (yield: 69%) of 7*β*-amino-3-[6-[2-(tert-butoxycarbonyl)aminoethylthiomethy]-7-oxo-7H-thieno[3,2-b]thiopyran-5-yl)thio-3-cephem-4-carboxylic acid benzhydryl ester as crude crystals.
(2) The obtained compound (290 mg, 0.38 mmol), (Z)-2-trityloxyimino-2-(2-tritylamino-5-chlorothiazol-4-yl)acetic acid (543 mg, 0.77 mmol) and dicyclohexylcarbodiimide (159 mg, 0.77 mmol) were used to conduct the procedure similar to that shown in (2) of Example 12 to obtain 159 mg (yield: 29%) of 7-[2-trityloxyimino-2-(2-tritylamino-5-chlorothiazol-4-yl)acetamido]-3-[6-[2-(tert-butoxycarbonyl)aminoethylthiomethyl]-7-oxo-7H-thieno[3,2-b]thiopyran-5-yl)thlo-3-cephem-4-carboxylic acid benzhydryl ester as brown froth.
   NMR(CDCl₃)δ: 1.42(9H, s), 3.26-3.32(3H, m), 3.66-3.73(3H, m), 4.61(2H, ABq, J=1.9Hz, 14.5Hz), 5.18(1H, d, J=5.0Hz), 6.15(1H, dd, J=5.0Hz, 9.2Hz), 6.97-7.06 (2H, m), 7.09(1H, d, J=5.3Hz), 7.18-7.40(40H, m), 7.88(1H, d, J=5.3Hz)
(3) This compound (145 mg, 0.10 mmol), anisole (229 mg, 2.00 mmol) and trifluoroacetic acid (1.95 g, 1.96 mmol) were used to conduct the procedure similar to that shown in (2) of Example 1 to obtain 25 mg (yield: 27%) of the titled compound as pale yellow powder.
   FAB-MS m/z: 692[M+H]⁺
   NMR(DMSO-d₆)δ: 3.19(2H, t, J=7.6Hz), 3.26-3.56(3H, m), 3.79(1H, d, J=17.2Hz), 4.72(2H, ABq, J=14.2Hz, 45.2Hz), 5.16(1H, d, J=5.3Hz), 5.74(1H, d, J=5.3Hz), 7.51(1H, d, J=5.3Hz), 8.21(1H, d, J=5.3Hz), 9.45(1H, d, J=8.6Hz)

### Example 18: Preparation of 7-[2-hydroxyimino-2-(2-amino-5-chlorothiazol-4-yl)acetamido]-3-(8-oxo-8H-thiopyrano[2,3-b]pyrazin-6-yl)thio-3-cephem-4-carboxylic acid (Compound 17)

(1) 7-Phenylacetoamido-3-trifluoromethanesulfonyloxy-3-cephem-4-carboxylic acid benzhydryl ester 1-oxide (894 mg, 1.38 mmol) and sodium 6-mercapto-8-oxo-8H-thiopyrano[2,3-b]pyrazine (1.39 mmol) dissolved in tetrahydrofuran were used to conduct the procedure similar to that shown in (1) of Example 11 to obtain 369 mg (yield: 39%) of 7-phenylacetamido-3-(8-oxo-8H-thiopyrano[2,3-b]pyrazin-6-yl)thio-3-cephem-4-carboxylic acid benzhydryl ester 1-oxide as yellow froth.
   NMR(CDCl₃)δ: 3.52(1H, d, J=17.8Hz), 3.63(2H, s), 3.96(1H, d, J=17.8Hz), 4.63(1H, brs), 6.17(1H, dd, J=5.0Hz, 9.0Hz), 6.92-6.99 (2H, m), 7.14-7.32(15H, m), 8.65(1H, brs), 8.84(1H, brs)
(2) This compound (365 mg, 0.52 mmol) and phosphorous trichloride (230 mg, 1.72 mmol) were used to conduct the procedure similar to (2) of Example 11 to obtain 261 mg (yield: 73%) of 7-phenylacetamido-3-[(8-oxo-8H-thiopyrano[2,3-b]pyrazin-6-yl)thio]-3-cephem-4-carboxylic acid benzhydryl ester as yellow froth.
   NMR(CDCl₃)δ: 3.43(1H, d, J=18.1Hz), 3.67(2H, ABq, J=15.5Hz), 3.73(1H, d, J=18.1Hz), 5.09(1H, d, J=5.0Hz), 5.98(1H, dd, J=5.0 Hz, 9.0Hz), 6.45(1H, d, J=9.0Hz), 6.98(1H, s), 6.99(1H, s), 7.09-7.39(15H, m), 8.71(1H, d, J=2.0Hz), 8.90(1H, d, J=2.0Hz)
(3) This compound (261 mg, 0.384 mmol), phosphorous pentachloride (160 mg, 0.769 mmol) and pyridine (60 mg, 0.769 mmol) were used to conduct the procedure similar to that shown in (1) of Example 12 to obtain 134 mg (yield: 62%) of *7β*-amino-3-(8-oxo-8H-thiopyrano[2,3-b]pyrazin-6-yl)thio-3-cephem-4-carboxylic acid benzhydryl ester as crude crystals.
(4) This compound (134 mg, 0.23 mmol), (Z)-2-trityloxyimino-2-(2-tritylamino-5-chlorothlazol-4-yl)acetic acid (337 mg, 0.47 mmol) and dicyclohexylcarbodiimide (97 mg, 0.47 mmol) were used to conduct the procedure similar to that shown in (2) of Example 12 to obtain 120 mg (yield: 40%) of 7-[2-trityloxyimino-2-(2-tritylamino-5-chlorothiazol-4-yl)acetamido]-3-(8-oxo-8H-thiopyrano[2,3-b]pyrazin-6-yl)thio-3-cephem-4-carboxylic acid benzhydryl ester as brown powder.
   NMR(CDCl₃)δ: 3.22(1H, d, J=18.0Hz), 3.71(1H, d, J=18.0Hz), 5.2 1(1H, d, J=5.0Hz), 6.22(1H, dd, J=5.0Hz, 9.0Hz), 6.64(1H, s), 7.01(1H, s), 7.03(1H, s), 7.05(1H, d, J=9.0Hz), 7.14-7.38(40H, m), 8.68(1H, d, J=2.0Hz), 8.88(1H, d, J=2.0Hz)
(5) This compound (120 mg, 0.096 mmol), anisole (210 mg, 1.924 mmol) and trifluoroacetic acid (1.968 g, 17.316 mmol) were used to conduct the procedure similar to that shown in (2) of Example 1 to obtain 20 mg (yield: 29%) of the titled compound as pale yellow powder.
   FAB-MS m/z: 598[M+H]⁺
   NMR(DMSO-d₆)δ: 3.61(1H, d, J=17.8Hz), 3.92(1H, d, J=17.8Hz), 5.32 (1H, d, J=5.0Hz), 5.95(1H, dd, J=5.0Hz, 9.0Hz), 7.15(1H, s), 7.21-7.36(2H, m), 8.88(1H, d, J=2.0Hz), 8.96(1H, d, J=2.0Hz), 9.64(1H, d, J=9.0Hz), 11.72(1H, s)

### Example 19: Preparation of 7-[2-hydroxyimino-2-(2-amino-5-chlorothiazol-4-yl)acetamido]-3-(6-hydroxymethyl-7-oxo-7H-thieno[3,2-b]thiopyran-5-yl)thio-3-cephem-4-carboxylic acid (Compound 18)

(1) 7-Phenylacetamido-3-(6-methoxymethyloxymethyl-7-oxo-7H-thieno[3,2-b]thiopyran-5-yl)thio-3-cephem-4-carboxylic acid benzhydryl ester (780 mg, 1.03 mmol), phosphorous pentachloride (429 mg, 2.06 mmol) and pyridine (163 mg, 2.06 mmol) were used to conduct the procedure similar to that shown (1) of Example 12 to obtain 427 mg (yield: 65%) of 7*β*-amino-3-(6-methoxymethyloxymethyl-7-oxo-7H-thieno[3,2-b]thiopyran-5-yl)thio-3-cephem-4-carboxylic acid benzhydryl ester as crude crystals.
(2) The obtained crude crystals (427 mg, 1.49 mmol), (Z)-2-trityloxyimino-2-(2-tritylamino-5-chlorothiazol-4-yl)acetic acid (1.45 g. 2.06 mmol) and dicyclohexylcarbodiimide (424 mg, 2.06 mmol) were used to conduct the procedure similar to that shown in (2) of Example 12 to obtain 392 mg (yield: 20%) of 7-[2-trityloxyimino-2-(2-tritylamino-5-chlorothiazol-4-yl)acetamido]-3-(6-methoxymethyloxymethyl-7-oxo-7H-thieno[3,2-b]thiopyran-5-yl)thio-3-cephem-4-carboxylic acid benzhydryl ester as crude crystals.
   NMR(CDCl₃)δ: 3.33(1H, d, J=17.8Hz), 3.34(3H, s), 3.63(1H, d, J=17.8 Hz), 4.65(2H, s), 4.73(2H, q, J=10.2Hz), 5.14(1H, d, J=5.0Hz), 6.14 (1H, dd, J=5.0Hz, 9.2Hz), 6.62(1H, brs), 6.98(1H, d, J=9.2Hz), 7.02 (1H, s), 7.08(1H, d, J=5.3Hz), 7.19-7.42(40H, m), 7.78(1H, d, J=5.3Hz)
(3) This compound (200 mg, 0.15 mmol), anisole (244 mg, 3.26 mmol) and trifluoroacetic acid (3.09 g, 27.12 mmol) were used to conduct the procedure similar to that shown in (2) of Example 1 to obtain 45 mg (yield: 40%) of the titled compound as pale yellow powder.
   FAB-MS m/z: 632[M+H]⁺
   NMR(DMSO-d₆)δ: 3.38(1H, d, J=17.2Hz), 3.68(1H, d, J=17.2Hz), 4.52(1H, d, J=11.2Hz), 4.65(1H, d, J=11.2Hz), 5.13(1H, d, J=5.0Hz), 5.74(1H, dd, J=5.0Hz, 8.2Hz), 7.47(1H, d, J=5.3Hz), 8.16(1H, d, J=5.3Hz), 9.41 (1H, d, J=8.2Hz)

### Example 20: Preparation of 7-[2-hydroxyimino-2-(2-amino-5-chlorothiazol-4-yl)acetamido]-3-(6-chloro-7-oxo-7H-1-thiopyrano[3,2-b]furan-5-yl)thio-3-cephem-4-carboxylic acid (Compound 19)

(1) 7-Phenylacetamido-3-(6-chloro-7-oxo-7H-1-thiopyrano[3,2-b]furan-5-yl)thio-3-cephem-4-carboxylic acid benzhydryl ester (780 mg, 1.03 mmol), phosphorous pentachloride (429 mg, 2.06 mmol) and pyridine (163 mg, 2.06 mmol) were used to conduct the procedure similar to that shown in (1) of Example 12 to obtain 427 mg (yield: 65%) of 7*β*-amino-3-(6-chloro-7-oxo-7H-1-thiopyrano[3,2-b]furan-5-yl)thio-3-cephem-4-carboxylic acid benzhydryl ester as crude crystals.
(2) The obtained crude crystals (427 mg, 1.49 mmol), (Z)-2-trityloxyimino-2-(2-tritylamino-5-chlorothiazol-4-yl)acetic acid (1.45 g, 2.06 mmol) and dicyclohexylcarbodiimide (424 mg, 2.06 mmol) were used to conduct the procedure similar to that shown in (2) of Example 12 to obtain 392 mg (yield: 20%) of 7-[2-trityloxyimino-2-(2-tritylamino-5-chlorothiazol-4-yl)acetamido]-3-(6-chloro-7-oxo-7H-1-thiopyrano[3,2-b]furan-5-yl)thio-3-cephem-4-carboxylic acid benzhydryl ester as crude crystals.
   NMR(CDCl₃)δ: 3.23(1H, d, J=17.8Hz), 3.66(1H, d, J=17.8Hz), 5.21(1H, d, J=5.3Hz), 6.18(1H, dd, J=5.3Hz, 9.2Hz), 6.57(1H, d, J=2.0Hz), 6.62 (1H, brs), 6.98(1H, d, J=9.2Hz), 7.01(1H, s), 7.13-7.38(40H, m), 7.78 (1H, d, J=2.0Hz)
(3) This compound (200 mg, 0.16 mmol), anisole (244 mg, 3.26 mmol) and trifluoroacetic acid (3.09 g, 27.12 mmol) were used to conduct the procedure similar to that shown in (2) of Example 1 to obtain 45 mg (yield: 40%) of the titled compound as pale yellow powder.
   FAB-MS m/z: 621[M+H]⁺
   NMR(DMSO-d₆)δ: 3.30(1H, d, J=17.2Hz), 3.73(1H, d, J=17.2Hz), 5.13(1H, d, J=5.0Hz), 5.68(1H, dd, J=5.0 Hz, 8.6Hz), 7.15(1H, d. J=2.0Hz), 7.23 (1H, brs), 8.24(1H, d, J=2.0Hz), 9.41(1H, d, J=8.9Hz), 11.63(1H, brs)

### Example 21: Preparation of 7-[2-hydroxyimino-2-(2-amino-5-chlorothiazol-4-yl)acetamido]-3-(7-oxo-7H-1-thiopyrano[3,2-b]furan-5-yl)thio-3-cephem-4-carboxylic acid (Compound 20)

(1) 7-Phenylacetamido-3-(7-oxo-7H-1-thiopyrano[3,2-b]furan-5-yl)thio-3-cephem-4-carboxylic acid benzhydryl ester (320 mg, 0.48 mmol) obtained in (1) of Example 6, phosphorous pentachloride (219 mg, 1.06 mmol) and pyridine (83 mg, 1.06 mmol) were used to conduct the procedure similar to that shown in (1) of Example 12 to obtain 152 mg (yield: 58%) of 7*β*-amino-3-(7-oxo-7H-1-thiopyrano[3,2-b]furan-5-yl)thio-3-cephem-4-carboxylic acid benzhydryl ester as crude crystals.
(2) The obtained crude crystals (152 mg, 0.28 mmol), (Z)-2-trityloxyimino-2-(2-tritylamino-5-chlorothiazol-4-yl)acetic acid (676 mg, 0.96 mmol) and dicyclohexylcarbodiimide (197 mg, 0.96 mmol) were used to conduct the procedure similar to that shown in (2) of Example 12 to obtain 193 mg (yield: 55%) of 7-[2-trityloxyimino-2-(2-tritylamino-5-chlorothiazol-4-yl)acetamido]-3-(7-oxo-7H-1-thiopyrano[3,2-b]furan-5-yl)thio-3-cephem-4-carboxylic acid benzhydryl ester as crude crystals.
   NMR(CDCl₃)δ: 3.20(1H, d, J=17.8Hz), 3.68(1H, d, J=17.8Hz), 5.15(1H, d, J=5.0Hz), 6.14(1H, dd, J=5.0Hz, 9.5Hz), 6.60(1H, brs), 6.66(1H, d, J=2.0Hz), 7.00(1H, s), 7.03(1H, d, J=9.5Hz), 7.14(1H, s), 7.19-7.41 (40H, m), 7.81(1H, d, J=2.0Hz)
(3) This compound (190 mg, 0.15 mmol), anisole (330 mg, 3.06 mmol) and trifluoroacetic acid (2.62 g, 23.04 mmol) were used to conduct the procedure similar to that shown in (2) of Example 1 to obtain 26 mg (yield: 25%) of the titled compound as pale yellow powder.
   FAB-MS m/z: 586[M+H]⁺
   NMR(DMSO-d₆)δ: 3.76(1H, d, J=17.2Hz), 4.05(1H, d, J=17.2Hz), 5.51(1H, d, J=5.0Hz), 6.09(1H, dd, J=5.0Hz, 8.6Hz), 7.32(1H, s), 7.39(1H, d, J=2.0Hz), 7.45(1H, brs), 8.50(1H, d, J=2.0Hz), 9.76(1H, d, J=8.6Hz), 11.93(1H, brs)

### Example 22: Preparation of 7-[2-hydroxyimino-2-(2-amino-5-chlorothiazol-4-yl)acetamido]-3-(2-chloro-4-oxo-4H-1-thieno[2,3-b]thiopyran-6-yl)thio-3-cephem-4-carboxylic acid (Compound 21)

(1) 7-Phenylacetamido-3-(2-chloro-4-oxo-4H-1-thieno[2,3-b]thiopyran-6-yl)thio-3-cephem-4-carboxylic acid benzhydryl ester (717 mg, 1.0 mmol) obtained in (1) of Example 7, phosphorous pentachloride (417 mg, 2.0 mmol) and pyridine (156 mg, 2.0 mmol) were used to conduct the procedure similar to that shown in (1) of Example 12 to obtain 7*β*-amino-3-(2-chloro-4-oxo-4H-1-thieno[2,3-b]thiopyran-6-yl)thio-3-cephem-4-carboxylic acid benzhydryl ester as crude crystals.
(2) The obtained crude crystals, (Z)-2-trityloxyimino-2-(2-tritylamino-5-chlorothiazol-4-yl)acetic acid (847.5 mg, 1.2 mmol) and dicyclohexylcarbodiimide (247.5 mg, 1.2 mmol) were used to conduct the procedure similar to that shown in (2) of Example 12 to obtain 410 mg (yield: 32%) of 7-[2-trityloxyimino-2-(2-tritylamino-5-chlorothiazol-4-yl)acetamido]-3-(2-chloro-4-oxo-4H-1-thieno[3,2-b]thiopyran-6-yl)thio-3-cephem-4-carboxylic acid benzhydryl ester as crude crystals.
   NMR(CDCl₃)δ: 3.19(1H, d, J=17.8Hz), 3.58(1H, d, J=17.8Hz), 5.14(1H, d, J=5.0Hz), 6.15(1H, dd, J=5.0Hz, 9.5Hz), 6.60(1H, s), 6.99(1H, s), 7.03(1H, s), 7.06(1H, d, J=9.5Hz), 7.10-7.41(40H, m), 7.52(1H, s)
(3) This compound (270 mg, 0.21 mmol), anisole (453 mg, 4.19 mmol) and trifluoroacetic acid (5.18 g, 45.4 mmol) were used to conduct the procedure similar to that shown in (2) of Example 1 to obtain 23 mg (yield: 14.6%) of the titled compound as pale yellow powder.
   FAB-MS m/z: 636[M+H]⁺
   NMR(DMSO-d₆)δ: 3.36(2H, d, J=17.5Hz), 3.83(1H, d, J=17.5Hz), 5.26(1H, d, J=5.0Hz), 5.90(1H, dd, J=5.0Hz, 8.6Hz), 7.06(1H, s), 7.56(1H, s), 9.53(1H, d, J=8.6Hz), 11.90(1H, s)

### Example 23: Preparation of 7-phenylacetamido-3-(3-bromo-7-oxo-7H-thieno[3,2-b]thiopyran-5-yl)thio-3-cephem-4-carboxylic acid (Compound 22)

7-Phenylacetamido-3-(3-bromo-7-oxo-7H-1-thieno[3,2-b]thiopyran-5-yl)thio-3-cephem-4-carboxylic acid benzhydryl ester (76.2 mg, 0.1 mmol), anisole (216 mg, 2.0 mmol) and trifluoroacetic acid (1.71 g, 15 mmol) were used to conduct the procedure similar to that shown in (2) of Example 1 to obtain 51 mg (yield: 87%) of the titled compound as brown powder.
FAB-MS m/z: 596[M+H]⁺
NMR(DMSO-d₆)δ: 3.55(1H, d, J=17.8Hz), 3.65(2H, s), 3.87(1H, d, J=17.8Hz), 5.24(1H, d, J=5.0Hz), 5.81(1H, dd, J-5.0 Hz, 8.0Hz), 7.13(1H, s), 7.20-7.30(5H, m), 8.49(1H, s), 9.29(1H, d, J=8.0Hz)

### Example 24: Preparation of 7-phenylacetamido-3-(2-bromo-4-oxo-4H-thieno[2,3-b]thiopyran-6-yl)thio-3-cephem-4-carboxylic acid (Compound 23)

7-Phenylacetamido-3-(2-bromo-4-oxo-4H-thieno[2,3-b]thiopyran-6-yl)thio-3-cephem-4-carboxylic acid benzhydryl ester (305 mg, 0.4 mmol), anisole (0.86 mg, 8.0 mmol) and trifluoroacetic acid (8.21 g, 72 mmol) were used to conduct the procedure similar to that shown in (2) of Example 1 to obtain 118 mg (yield: 50%) of the titled compound as yellow powder.
FAB-MS m/z: 596[M+H]⁺
NMR(acetone-d₆)δ: 3.65(1H, d, J=18.0Hz), 3.68(2H, ABq, J=14.0Hz), 3.93(1H, d, J=18.0Hz), 5.27(1H, d, J=5.0Hz), 5.94(1H, dd, J=5.0Hz, 9.0Hz), 7.05(1H, s), 7.20-7.40(5H, m), 7.65(1H, s), 8.20(1H, d, J=9.0Hz)

### Example 25: Preparation of 7-[2-hydroxyimino-2-(2-amino-5-chlorothiazol-4-yl)acetamido]-3-(4-oxo-4H-1-benzothiopyran-2-yl)thio-3-cephem-4-carboxylic acid (Compound 24)

(1) 7-Phenylacetamido-3-(4-oxo-4H-1-benzothiopyran-2-yl)thio-3-cephem-4-carboxylic acid benzhydryl ester (5.96 g, 8.8 mmol) obtained in (1) of Example 4, phosphorous pentachloride (3.67 g, 17.6 mmol) and pyridine (1.34 g, 17.6 mmol) were used to conduct the procedure similar to that shown (1) of Example 12 to obtain 3.23 g (yield: 66%) of 7*β*-amino-3-(4-oxo-4H-1-benzothiopyran-2-yl)thio-3-cephem-4-carboxylic acid benzhydryl ester as crude crystals.
(2) The obtained crude crystals (3.23 g, 5.78 mmol), (Z)-2-trityloxyimino-2-(2-tritylamino-5-chlorothiazol-4-yl)acetic acid (4.91 g, 6.95 mmol) and dicyclohexylcarbodiimide (1.43 g, 6.95 mmol) were used to conduct the procedure similar to that shown in (2) of Example 12 to obtain 1.27 g (yield: 18%) of 7-[2-trityloxyimino-2-(2-tritylamino-5-chlorothiazol-4-yl)acetamido]-3-(4-oxo-4H-1-benzothiopyran-2-yl)thio-3-cephem-4-carboxylic acid benzhydryl ester as crude crystals.
   NMR(CDCl₃)δ: 3.24(1H, d, J=17.8Hz), 3.65(1H, d, J=17.8Hz), 5.17(1H, d, J=5.0Hz), 6.16(1H, dd, J=5.0 Hz, 9.0Hz), 6.59(1H, s), 6.98(1H, s), 7.01-7.04(2H, d+s, J=9.0Hz), 7.20-7.60(43H, m), 8.40-8.50(1H, m)
(3) This compound (249 mg, 0.20 mmol), anisole (432 mg, 4.0 mmol) and trifluoroacetic acid (4.1 g, 36.0 mmol) were used to conduct the procedure similar to that shown in (2) of Example 1 to obtain 13.2 mg (yield: 10%) of the titled compound as pale yellow powder.
   FAB-MS m/z: 596[M+H]⁺
   NMR(DMSO-d₆)δ: 3.57(1H, d, J=17.8Hz), 3.89(1H, d, J=17.8Hz), 5.30(1H, d, J=5.0Hz), 5.92(1H, dd, J=5.0Hz, 9.0Hz), 6.98(1H, s), 7.20-7.40(2H, m), 7.60-7.70(2H, m), 7.81(1H, d, J=8.0Hz), 8.31(1H, d, J=8.0Hz), 9.62(1H, d, J=9.0Hz), 11.7(1H, s)

### CAPABILITY OF EXPLOITATION IN INDUSTRY

The cephem compound according to the present invention has excellent antibacterial activity against MRSA and vancomycin-resistant E. faecalis and therefore is applicable to treatment of infections due to MRSA and other pathogenic bacteria.

## Claims

1. Cephem derivatives or pharmaceutically acceptable salts thereof represented by the formula I: 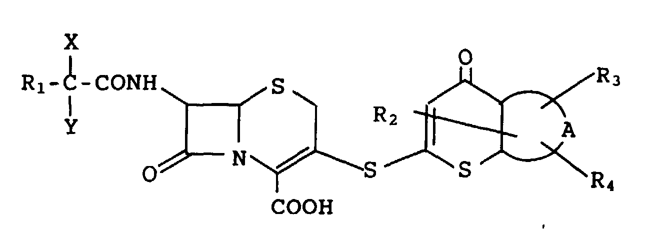 wherein 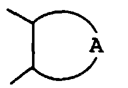 represents benzene ring, pyridine ring, pyrazine ring or 5-membered aromatic heterocycle (having one oxygen or sulfur atom as ring-constituting atom);
X and Y respectively represent hydrogen atoms or CXY represents C=N-OH;
R₁ represents phenyl, thienyl, thiazolyl (which may be substituted with amino group or halogen atom); and
R₂, R₃ and R₄ respectively represent hydrogen atoms, halogen, hydroxy C₁-C₆ alkyl, isothiuronium C₁-C₆ alkyl, amino C₁-C₆ alkyl or amino C₁-C₆ alkylthiomethyl, there being no R₄ where 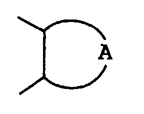 represents 5-membered aromatic heterocycle.

2. The compound according to claim 1 wherein 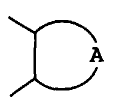 is benzene ring, X and Y are respectively hydrogen atom and R₁ is phenyl.

3. The compound according to claim 1 wherein 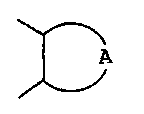 is benzene ring, CXY is C=N-OH and R₁ is thiazolyl which may be substituted with amino or chloro.

4. The compound according to claim 1 wherein 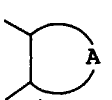 is furan ring, X and Y are respectively hydrogen atoms, and R₁ is phenyl.

5. The compound according to claim 1 wherein 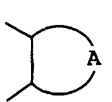 is furan ring, CXY is C=N-OH and R₁ is thiazolyl which may be substituted with amino or chloro.

6. The compound according to claim 1 wherein 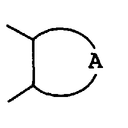 is thiophene ring.

7. The compound according to claim 1 wherein 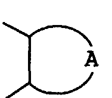 is thiophene ring and X and Y are respectively hydrogen atoms.

8. The compound according to claim 1 wherein 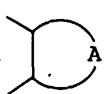 is thiophene ring, X and Y are respectively hydrogen atoms and R₁ is phenyl or thienyl.

9. The compound according to claim 1 wherein 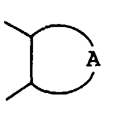 is thiophene ring and CXY is C=N-OH.

10. The compound according to claim 1 wherein 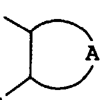 is thiophene ring, CXY is C=N-OH and R₁ is thiazolyl which may be substituted with amino or chloro.

11. Synthetic intermediates of cephem derivatives represented in claim 1, said synthetic intermediates having the formula II: 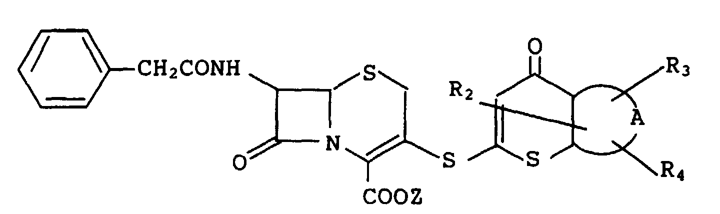 wherein 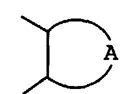 represents benzene ring, pyridine ring, pyrazine ring or 5-membered aromatic heterocycle (having one oxygen or sulfur atom as ring-constituting atom); and
R₂, R₃ and R₄ respectively represent hydrogen atoms, halogen, hydroxy C₁-C₆ alkyl, isothiuronium C₁-C₆ alkyl, amino C₁-C₆ alkyl or amino C₁-C₆ alkylthiometyl and Z represents a protective group for carboxyl.
